# EUROPEAN PATENT SPECIFICATION

(11) **EP 1 198 585 B1**
(45) Date of publication and mention of the grant of the patent: **13.06.2007**
(21) Application number: 00944977.8
(22) Date of filing: 28.06.2000
(51) Int. Cl.: C12Q 1/68

(54) **Method for characterizing a biological condition using calibrated gene expression profiles**
Verfahren zum Charakterisieren von biologischen Bedingungen, die kalibrierte Genexpressionsprofile verwenden
Procédé pour caractériser un état biologique employant profils calibrés d'expression génique

(30) Priority: 28.06.1999 US 141542 P; 07.04.2000 US 195522 P
(43) Date of publication of application: 24.04.2002
(60) Divisional application: 06019765.4
(73) Proprietor: Source Precision Medicine, Inc., Boulder, CO 80301 (US)
(72) Inventor: TRYON, Victor, Loveland, CO 80537 (US); BEVILACQUA, Michael, P., Boulder, CO 80301 (US); BANKAITIS-DAVIS, Danute, M., Longmont, CO 80503 (US); CHERONIS, John, Conifer, CO 80433 (US)
(74) Representative: Crump, Julian Richard John
(86) International application number: PCT/US2000/017846
(87) International publication number: WO 2001/025473

(56) References cited:
- WO-A1-94/23023
- WO-A1-98/24935
- WO-A1-99/04251
- WO-A1-99/11822

## Description

### Technical Field

A method is provided for identifying reproducible patterns of variation of gene expression that are informative by virtue of the degree of variation observed in a calibrated data set. The variations may be correlated with other non- genetic indications such as clinical indicators (for humans) of a traditional nature but are not required per se to be causative.

### Background Art

There has been substantial discussion including congressional hearings concerning medical errors. One source of medical errors include errors with medications. Upwards of 98,000 hospitalized patients annually have been documented to be victims of medication errors (Statement of the American Pharmaceutical Association to the Senate Appropriations Committee Labor, health and Human Services Education Subcommittee Hearing on Medical Errors December 13,1999). These errors include problems arising from drug interactions for a particular patient taking more than one drug, problems concerning the response of an individual to a particular drug and incorrect medication for a particular condition. Medical errors further arise as a result of misdiagnosis. This may occur as a result of insensitive diagnostic techniques or a wide range of interpersonal variability in the manner in which a clinical state is manifest. At present, there are few tools available for optimizing prognosis, diagnosis and treatment of a medical condition taking into account the particular phenotype and genotype of an individual.

There has been increasing interest in herbal drugs or nutraceuticals. These are often grown in developing countries and undergo little or no quality control. It is frequently the case that one batch of a nutraceutical may be effective, there is no assurance that a second batch will be effective Moreover, analysis of nutraceuticals is problematic because these drugs are complex mixtures in which little is known with respect to the active agent.

All new therapeutic agents require some form of clinical trials. It is known that a drug for treating tumor that is tested in a clinical trial using standard recruiting techniques for patients, may in fact show only limited efficacy. If the beneficial effect observed in a clinical population is too small, the drug will not receive approval by the Food and Drug Administration for use in the population at large. However, the small beneficial effect observed may in fact be an artifact of the clinical trial design or the clinical endpoint in the population of patients. It would be desirable to have criteria for screening patients as they enter a clinical trial to ensure that the beneficial effect of a drug if it exists may be detected and quantified.

WO 98/24935 A1 discloses methods for identifying specific disease state markers that are expressed in peripheral lymphocytes of patents in response to a disease state, at a different level than such markers are expressed in the peripheral blood of a normal subject. The use of relative quantitative RT-PCR (reverse transcriptase PCR) is disclosed for identifying alternatively spliced forms of IL-8 mRNA that are differentially expressed between normal individuals and individuals with metastatic prostate cancer. Such alternatively spliced forms of IL-8 mRNA may be used as diagnostic biomarkers. In some embodiments measurement of serum IL-8 gene product may be combined with other markers of prostate disease, such as PSA, PAP, HK2, PSP₉₄ and PSMAₓ.

### Summary of the Invention

According to the present invention there is provided a method for evaluating a biological condition of a subject, based on a first sample obtained from the subject, the sample providing a source of RNAs; characterised in that the method comprises:
(a) deriving from the first sample a first profile data set, which first profile data set comprises 3 or more members, each member being a relative quantitative measure of the amount in said first sample of a distinct transcribed RNA constituent in a panel of constituents selected such that measurement of said constituents enables evaluation of said biological condition, the relative quantitation of mRNA in said sample being determined by performing quantitative PCR on RNA extracted from the sample with a defined efficiency of amplification for the target transcripts and determining the difference in threshold cycles (delta C_{T}) between a size marker and the target transcripts of interest; and
(b) producing a calibrated profile data set for the panel, each member of the calibrated profile data set being a function of a corresponding member of the first profile data set and a corresponding member of a baseline profile data set for the panel; wherein the calibrated profile data set represents a set of values in which a pattern of variation correlates in a reproducible manner with a particular condition.

In a preferred embodiment, a method is provided for evaluating a biological condition affected by an agent, the method including: obtaining, from a target population of cells to which the agent has been administered, a sample having at least one of RNAs and proteins; deriving from the sample a first profile data set, the first profile data set including a plurality of members, each member being a quantitative measure of the amount of a distinct RNA or protein constituent in a panel of constituents selected so that measurement of the constituents enables measurement of the biological condition; and producing a calibrated profile data set for the panel, wherein each member of the calibrated profile data set is a function of a corresponding member of the first profile data set and a corresponding member of a baseline profile data set for the panel, the calibrated profile data set providing a measure of the biological condition as affected by the agent.

In a preferred embodiment, a method is provided for evaluating the effect on a biological condition by a first agent in relation to the effect by a second agent, including: obtaining, from first and second target populations of cells to which the first and second agents have been respectively administered, first and second samples respectively, each sample having at least one of RNAs and proteins; deriving from the first sample a first profile data set and from the second sample a second profile data set, the profile data sets each including a plurality of members, each member being a quantitative measure of the amount of a distinct RNA or protein constituent in a panel of constituents selected so that measurement of the constituents enables measurement of the biological condition; and producing for the panel a first calibrated profile data set and a second profile data set, wherein (i) each member of the first calibrated profile data set is a function of a corresponding member of the first profile data set and a corresponding member of a first baseline profile data set for the panel, and (ii) each member of the second calibrated profile data set is a function of a corresponding member of the second profile data set and a corresponding member of a second baseline profile data set for the panel, the calibrated profile data sets providing a measure of the effect by the first agent on the biological condition in relation to the effect by the second agent.

In a preferred embodiment, a method of conducting a clinical trial of an agent, is provided, including: causing the blind administration of a selected one of a placebo and the agent to each candidate of a pool of subjects; and using quantitative gene expression to monitor an effect of such administration.

In a preferred embodiment, a digital storage medium is provided on which is stored a computer readable calibrated profile data set, wherein: the calibrated profile data set relates to a sample having at least one of RNAs and proteins derived from a target cell population to which an agent has been administered; the calibrated profile data set includes a first plurality of members, each member being a quantitative measure of a change in an amount of a distinct RNA or protein constituent in a panel of constituents selected so that measurement of the constituents enables measurement of a biological condition as affected by administration of the agent.

In a preferred embodiment, a digital storage medium is provided on which is stored a plurality of records *Rᵢ* relating to a population of subjects, each record *Rᵢ* corresponding to a distinct instance *Pᵢ* of a computer readable profile data set *P* wherein: each instance *Pᵢ* of the profile data set *P* relates to a distinct sample derived from a subject, the sample having at least one of RNAs and proteins; the profile data *P* set includes a plurality of members *M*ⱼ, each member Mⱼ being a quantitative measure of the amount of a distinct RNA or protein constituent in a panel of constituents selected so that measurement of the constituents enables measurement of a biological condition; each record *Rᵢ* includes, for each member *Mᵢⱼ* of a corresponding distinct instance *Pᵢ* of the profile data set *P*, a value corresponding to the value of the member *Mᵢⱼ*; and each record *Rᵢ* also includes a reference to a characteristic of the subject relative to the record, the characteristic being at least one of age group, gender, ethnicity, geographic location, diet, medical disorder, clinical indicator, medication, physical activity, body mass, and environmental exposure.

In a preferred embodiment, a digital storage medium is provided on which is stored a large number of computer readable profile data sets, wherein each profile data set relates to a sample derived from a target cell population to which has been administered an agent, the sample having at least one of RNAs and proteins; each profile data set includes a plurality of members, each member being a quantitative measure of the amount of a distinct RNA or protein constituent in a panel of constituents selected so that measurement of the constituents enables measurement of a biological condition; and the panel is the same for all profile data sets.

In a preferred embodiment of the invention, a method is provided for evaluating a biological condition of a subject, based on a sample from the subject, the sample having at least one of RNAs and proteins, the method including: deriving from the sample a first instance of a profile data set, the profile data set including a plurality of members, each member being a quantitative measure of the amount of a distinct RNA or protein constituent in a panel of constituents selected so that measurement of the constituents enables measurement of the biological condition; and producing a first instance of a calibrated profile data set for the panel, wherein each member of an instance of the calibrated profile data set is a function of a corresponding member of an instance of the profile data set and a corresponding member of an instance of a baseline profile data set for the panel, the calibrated profile data set providing a measure of the biological condition of the subject; accessing data in a condition database, the condition database having a plurality of records relating to a population of subjects, each record corresponding to a distinct instance of the calibrated profile data set; and evaluating the first instance of the calibrated profile data set in relation to data in the condition database.

In a preferred embodiment of the invention, a method is provided of displaying quantitative gene expression analysis data associated with measurement of a biological condition, the method including: identifying a first profile data set pertinent to the gene expression analysis data, the first profile data set including a plurality of members, each member being a quantitative measure of the amount of a distinct RNA or protein constituent in a panel of constituents selected so that measurement of the constituents enables measurement of the biological condition; producing a calibrated profile data set for the panel, wherein each member of the calibrated profile data set is a function of a corresponding member of the first profile data set and a corresponding member of a baseline profile data set for the panel, the calibrated profile data set providing a measure of the biological condition of the subject; and displaying the calibrated profile data set in a graphical format.

A preferred embodiment is directed to a descriptive record of a change in a biological condition, that includes: a first set of numerical gene expression values for a panel of gene loci, each value in the set corresponding to a single gene locus in a panel of gene loci, the set of values forming a profile data set for a population of cells subjected to a first biological condition; a second set of numerical gene expression values for the panel of gene loci, each value in the set corresponding to a single gene locus, the set of values forming a baseline profile data set for a second population of cells subjected to a second biological condition, the second set of values optionally being an average for multiple gene expression values from multiple populations of cells for each locus in the panel; and a third set of numbers corresponding to the ratio of the first set of values and the second set of values with respect to each gene locus in the panel, the third set being a calibrated profile data set; the profile data set and the calibrated profile data set being descriptive of the first biological condition with respect to the second biological condition.

In a preferred embodiment, a method for diagnosing a biological condition of a subject is provided that includes: obtaining a sample from a subject; subjecting a population of cells to the sample and determining the presence of a first biological condition with respect to a second biological condition according to any of the above claims.

In a preferred embodiment, a method is provided for diagnosing a susceptibility for a biological condition in a subject, that includes obtaining a sample from the subject; creating a descriptive record, according to the above, wherein the baseline set of values is an average of second values contained in a library of descriptive records for the second biological condition; the library containing a plurality of descriptive records grouped according to a predetermined biological condition; comparing the calibrated profile data set of the subject with the library of calibrated profile data sets and diagnosing the susceptibility of the subject.

In a preferred embodiment, a method is provided for monitoring the progress of a biological condition, including: creating a plurality of descriptive records, according to the above; wherein each set of first values is determined at preselected time intervals with respect to the first record; comparing each calibrated profile data set with a library of calibrated profile data sets, the plurality of calibrated profile data sets being grouped according to a predetermined biological condition; and determining the progress of the biological condition with respect to gene expression.

In a preferred embodiment, a method is provided for establishing the biological activity of a composition, including: selecting a population of cells; subjecting the cells to the composition; and determining the record according to the above description using a standardized baseline profile data set for the biological condition.

In a preferred embodiment, a method is provided for determining which therapeutic agent from a choice of a plurality of therapeutic agents to administer to a subject so as to change a biological condition in a subject from a first biological condition to a second biological condition; including: subjecting a sample from the subject to each of a plurality of therapeutic agents; determining a descriptive record for each of the samples according to any of the above described methods, comparing each of the calibrated profile data sets to a library of calibrated profile data sets, the library of calibrated data sets being grouped according to a predetermined biological condition; and determining which of the therapeutic agents is capable of changing the first biological condition in the subject to the second biological condition in the subject.

In a preferred embodiment, a method is provided for characterizing the biological effectiveness of a single batch of a composition produced by a manufacturing process, comprising: providing a fingerprint or signature profile according to any of the above methods; and labeling the batch of the composition by placing the fingerprint (signature profile) on each container in the batch.

In a preferred embodiment, a method is provided for accessing biological information on a digital storage medium as described above, including: making the information available to a user.

In a preferred embodiment, a method is provided for consumer evaluation of a product, wherein the consumer evaluation is dependent on a signature profile, including: identifying the product using the signature profile.

In a preferred embodiment, a computer program product is provided for evaluating a biological condition of a subject or for evaluating a biological condition resulting from the use of an agent, including a computer usable medium having computer readable program code thereon, the computer program code; including: a program code for classifying a sample from the subject or the agent for an identifiable record; a program code for deriving a first data set, the first profile data set including a plurality of members, each member being a quantitative measure of the amount of a distinct RNA or protein constituent in a panel of constituents selected so that measurement of the constituents enables measurement of the biological condition; the profile data set being stored in the record; and a program code for optionally producing a calibrated profile data set for the panel, for storage in the record, each member of the calibrated profile data set being a function of a corresponding member of the first profile data set and a corresponding member of a baseline profile data set for the panel, the calibrated profile data set providing a measure of the biological condition of the subject.

### Brief Description of the Drawings

The foregoing features of the invention will be more readily understood by reference to the following detailed description, taken with reference to the accompanying drawings, in which:

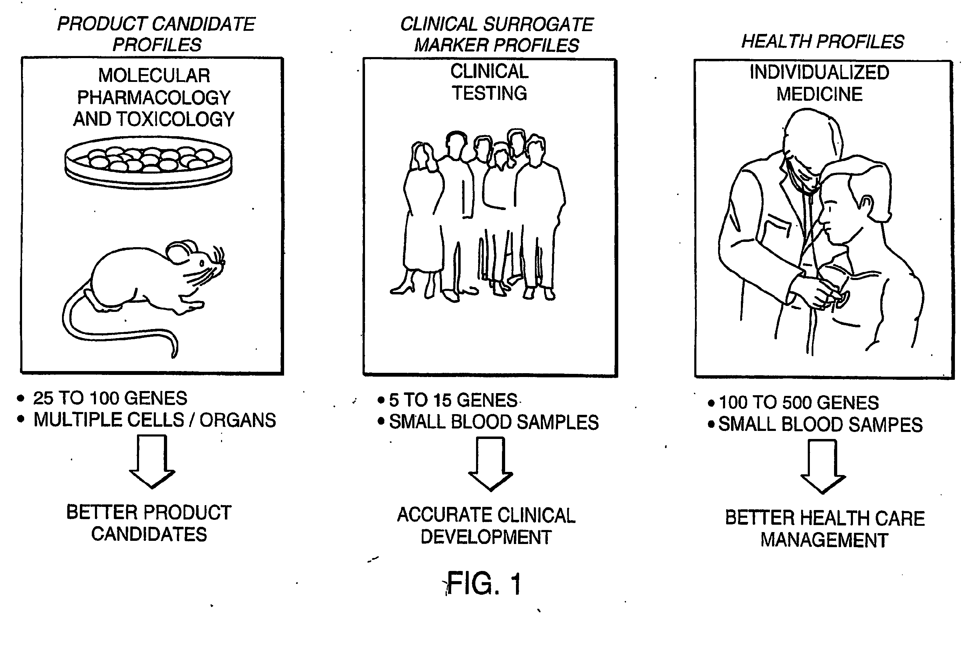

Figure 1 is a diagram showing the flow of information from data acquired in molecular pharmacology and toxicology, clinical testing, and use of the data for the application to individualized medicine.

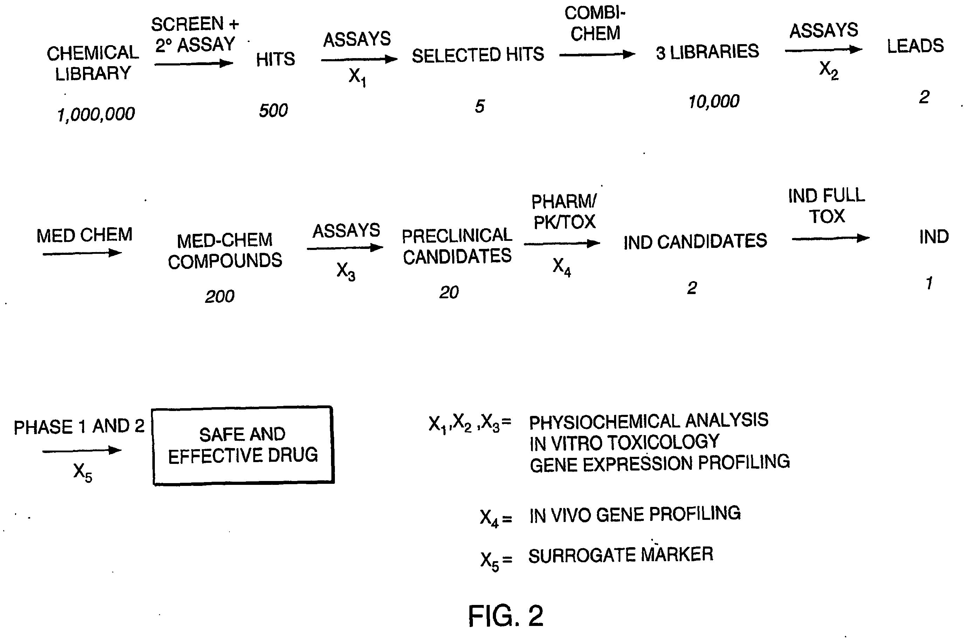

Figure 2 is a diagram showing the drug discovery pathway of new compounds from early leads to likely drug candidates. Although calibrated profile data sets are indicated at the pre-clinical step, gene expression data can be acquired and is useful at any of the stages shown. IND refers to investigative new drug and refers to an early stage in regulatory review.

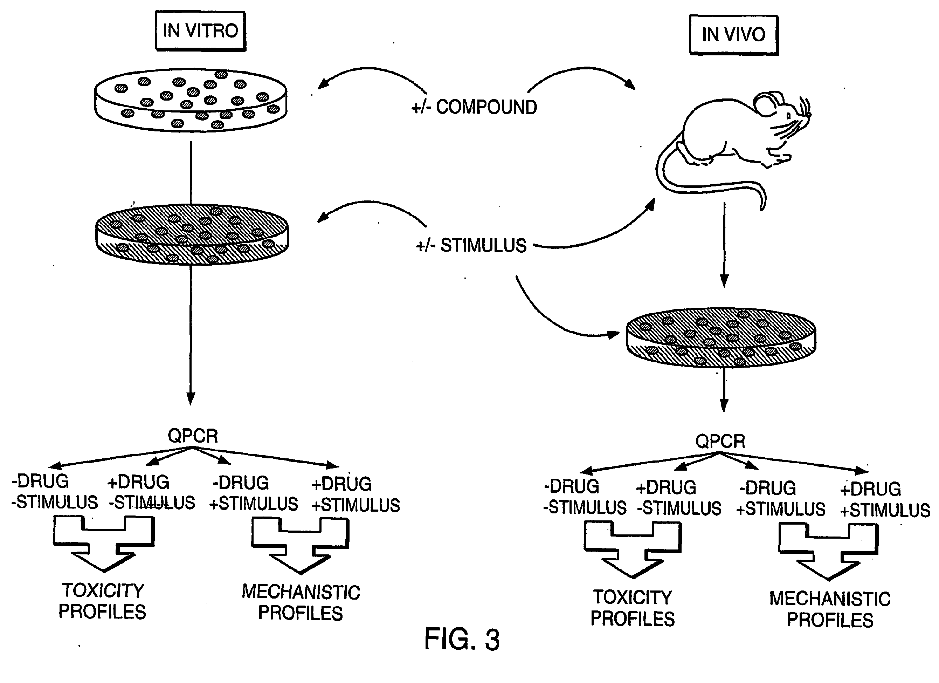

Figure 3 is a diagram presenting a comparison of *in vivo* and *in vitro* protocols for forming calibrated profile data sets for rapidly assessing product candidate toxicity and efficacy in accordance with several embodiments of the present invention.

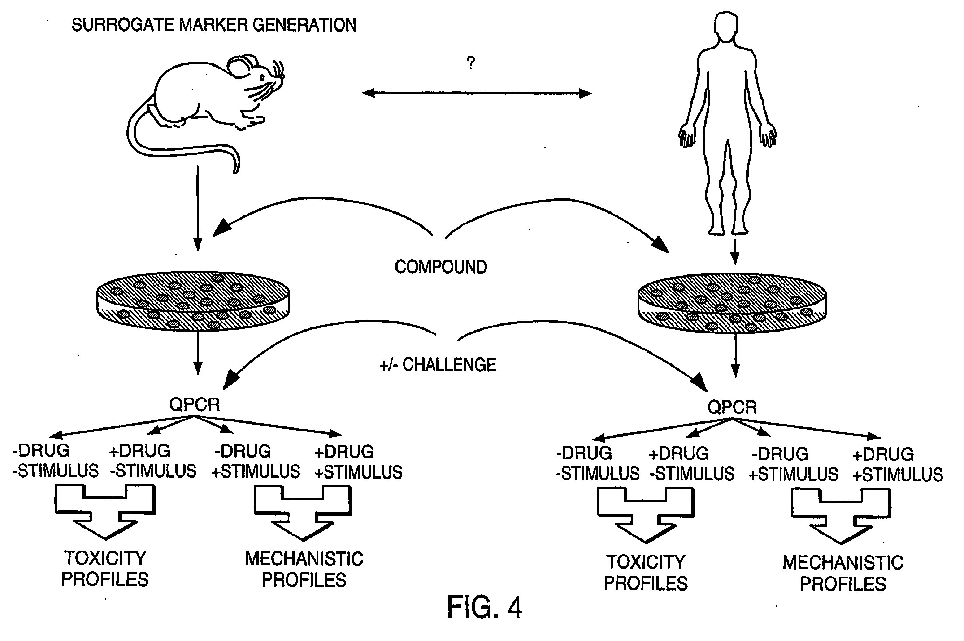

Figure 4 is a diagram showing the application of gene expression profiling as a guide to pre-clinical and clinical studies in accordance with an embodiment of the present invention.

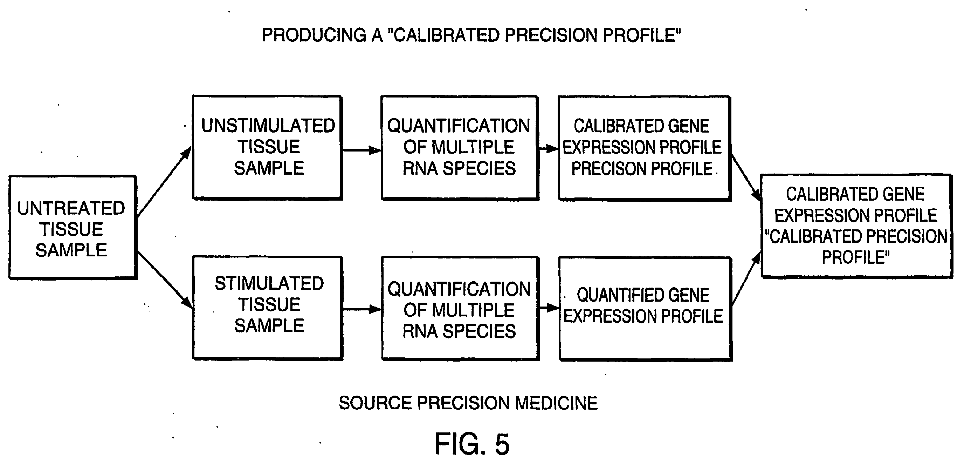

Figure 5 is a diagram showing a method in accordance with an embodiment of the present invention for obtaining profile data in the absence of a stimulus and in the presence of a stimulus.

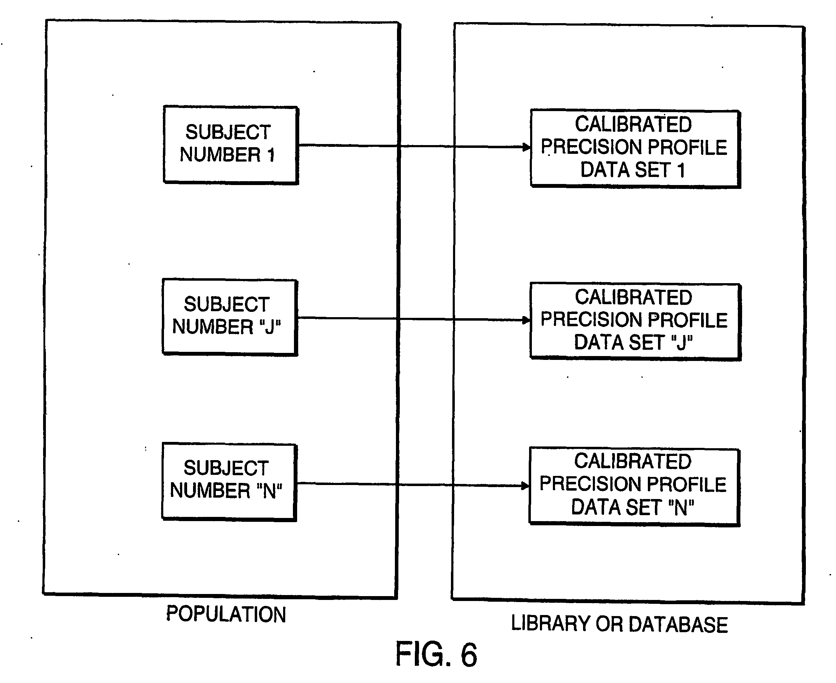

Figure 6 is a diagram showing the creation of a library of profile data associated with a plurality of subjects in accordance with an embodiment of the present invention.

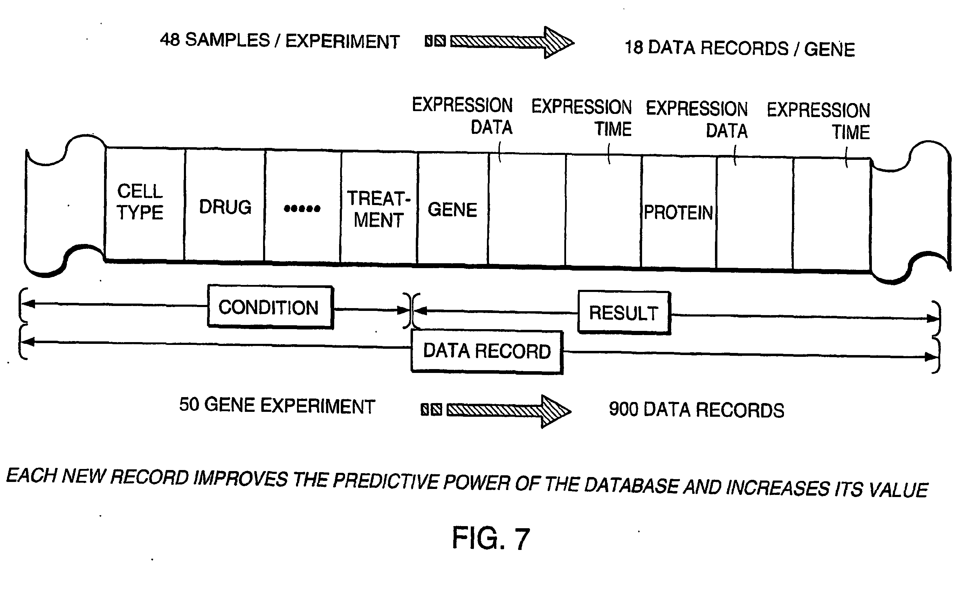

Figure 7 is a diagram illustrating the structure of a profile data record in accordance with an embodiment of the present invention.

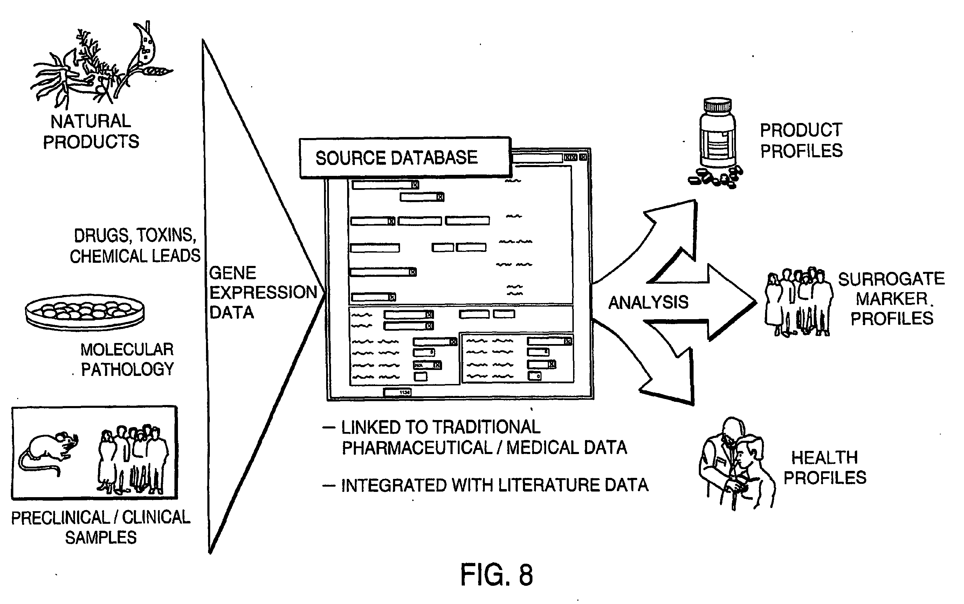

Figure 8 is a diagram illustrating a data entry screen for a data record of the type shown in Figure 7 and typical contexts in which data records may be compiled in accordance with embodiments of the present invention.

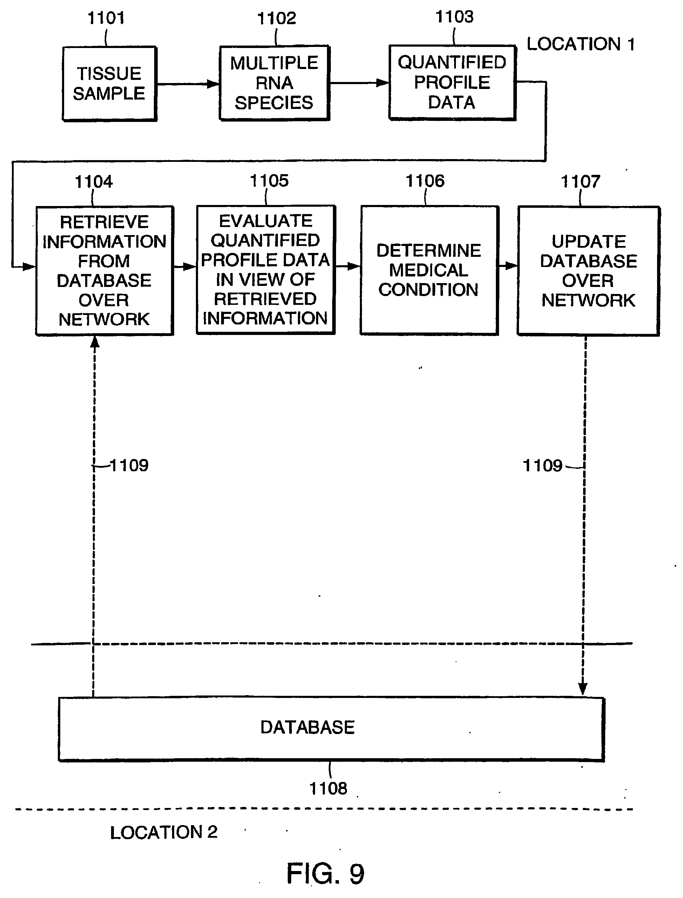

Figure 9 shows an embodiment of the present invention in which profile data, in either the raw or calibrated form, is evaluated using data from a database that is remotely accessed over a network.

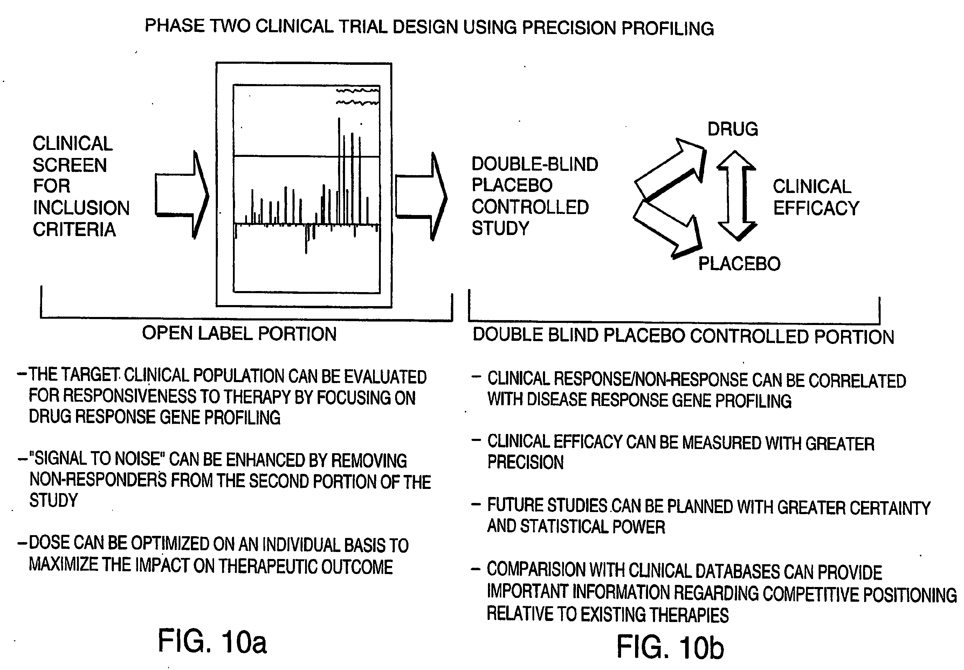

Figure 10 shows a schematic of a phase two clinical trial that utilizes gene expression profiling (a) The right hand panel (b) indicates that the same information may be used in Phase IV or post marketing studies to compare the efficacy of already approved and marketed drugs or to guide the marketing of such therapies; to guide the choice of therapy for an individual subject or population from within a class of appropriate compounds.

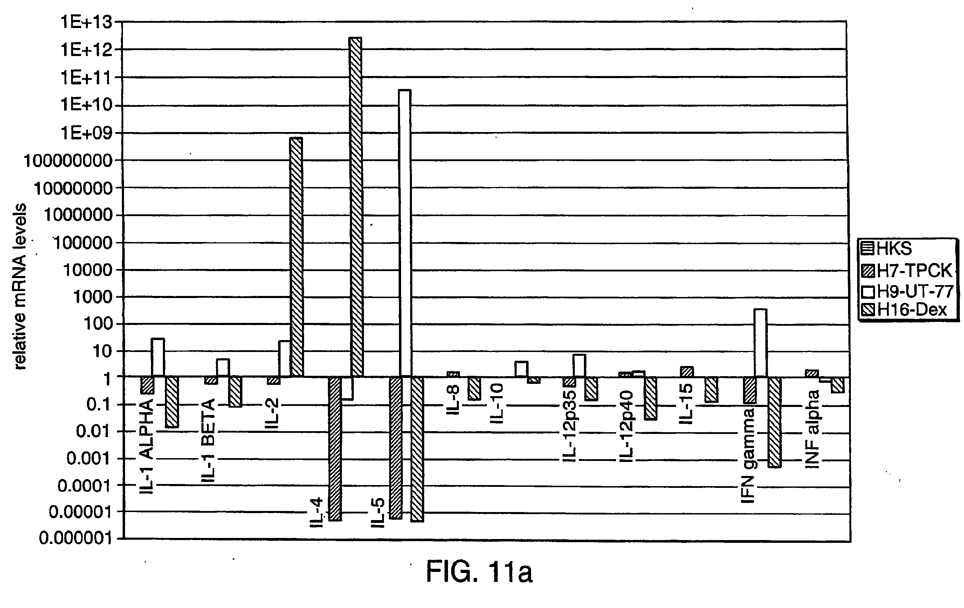

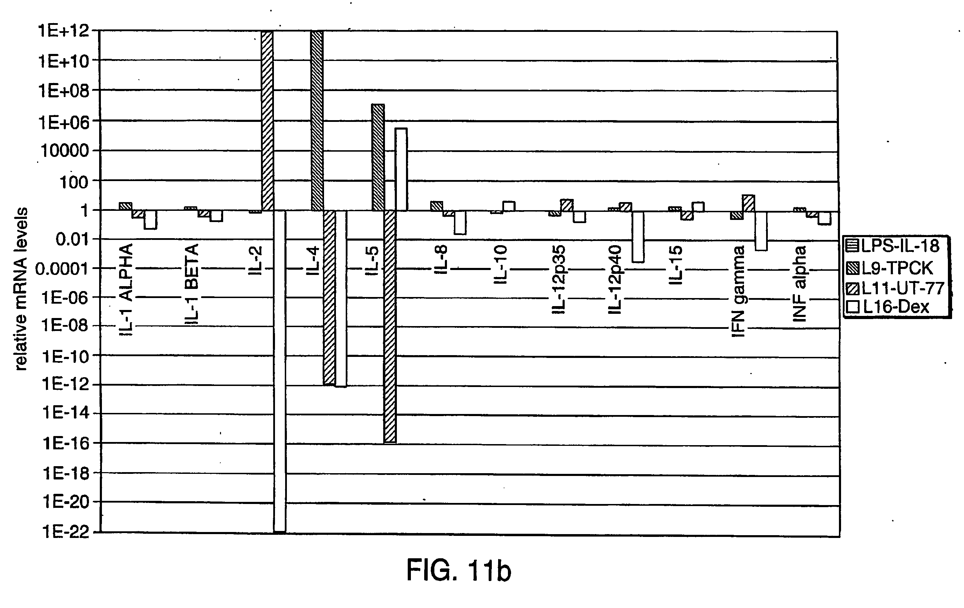

Figure 11 is a bar graph that shows a graphical representation in the form of a histogram representing calibrated profile data sets based on quantitative expression of RNA in cells of a whole blood sample using a panel of 12 constituents where each constituent corresponds to a unique gene locus. (a) The blood sample is stimulated *ex vivo* with heat killed staphylococci are further exposed H7-TPCK, H9-UT-77, or H16-Dex as indicated. The baseline profile data set is a blood sample stimulated *ex vivo* (*in vitro*) with heat killed staphylococci (b) The blood sample is stimulated *ex vivo* with lipopolysaccharide (LPS) and is then further exposed to compounds H7 TPCK, H9-UT-77, or H16-Dex as indicated.

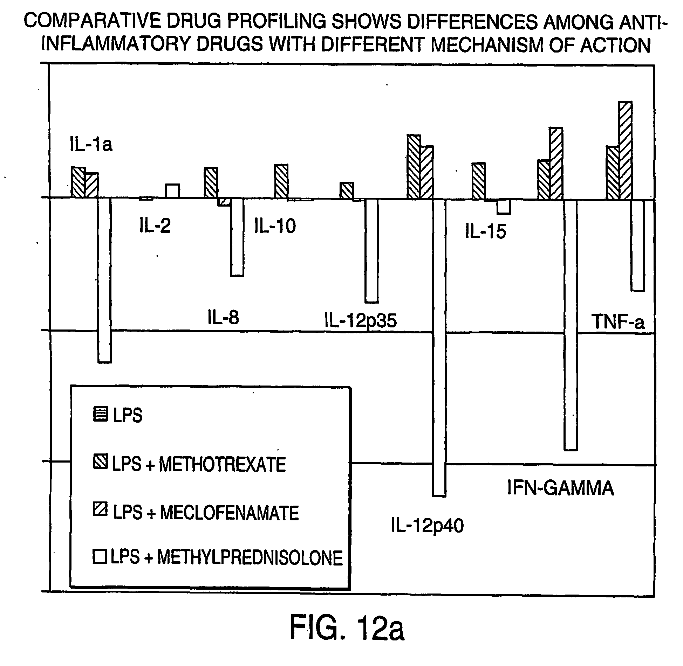

Figure 12 is a bar graph with a logarithmic axis that shows a graphical representation of calibrated profile data sets for whole blood stimulated *ex vivo* with lipopolysaccharide (LPS), using a panel of 9 constituents, each constituent corresponding to a gene locus encoding the gene products indicated, the blood being further exposed to anti-inflammatory agents: methotrexate, meclofenamate and methylprednisolone. The baseline profile data set is derived from LPS stimulated (but otherwise untreated) cells.

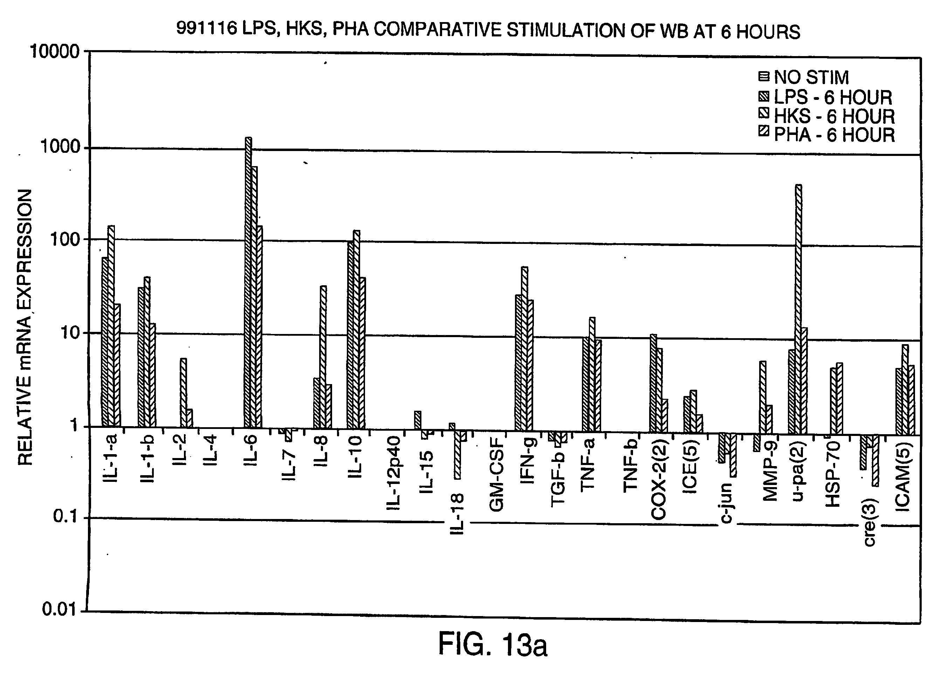

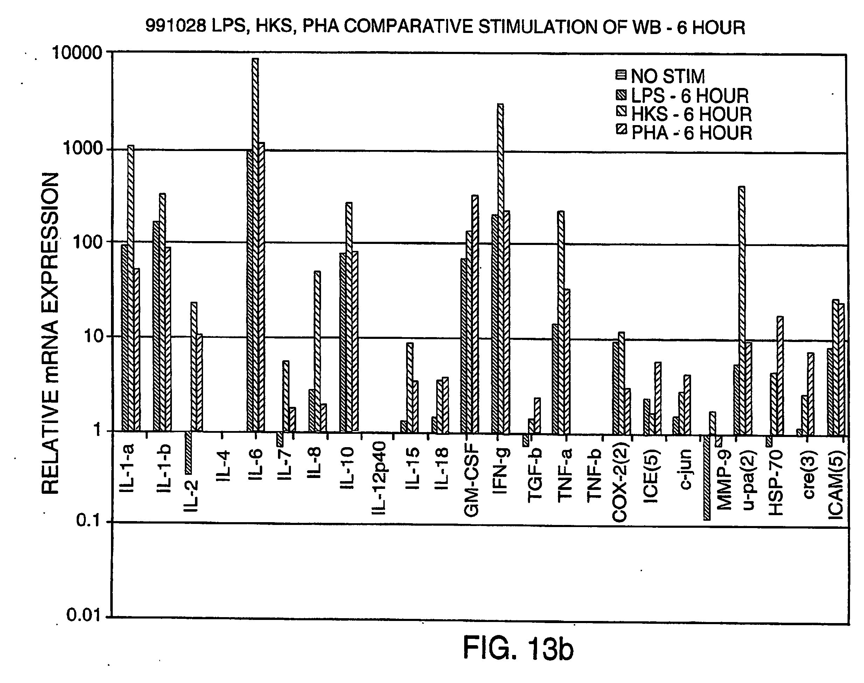

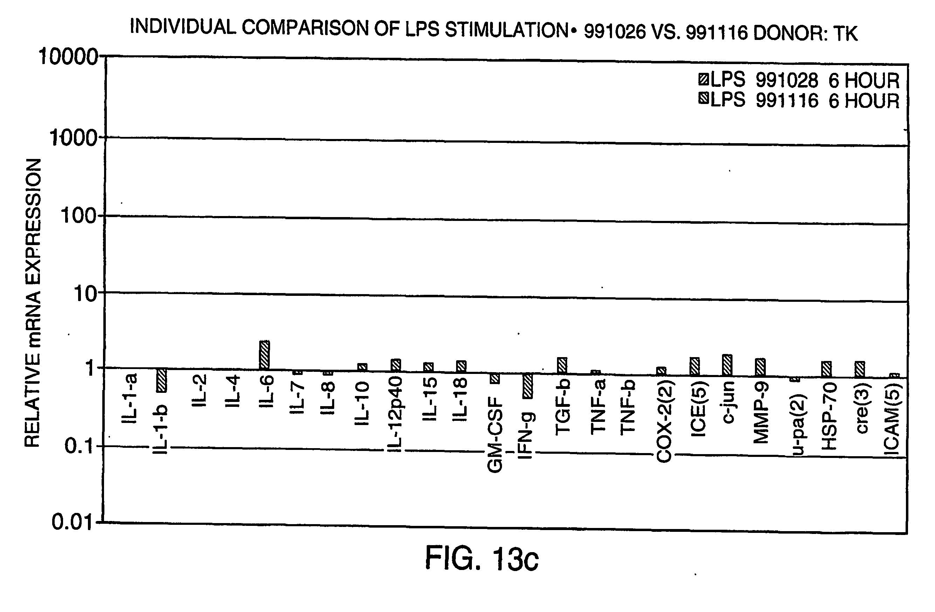

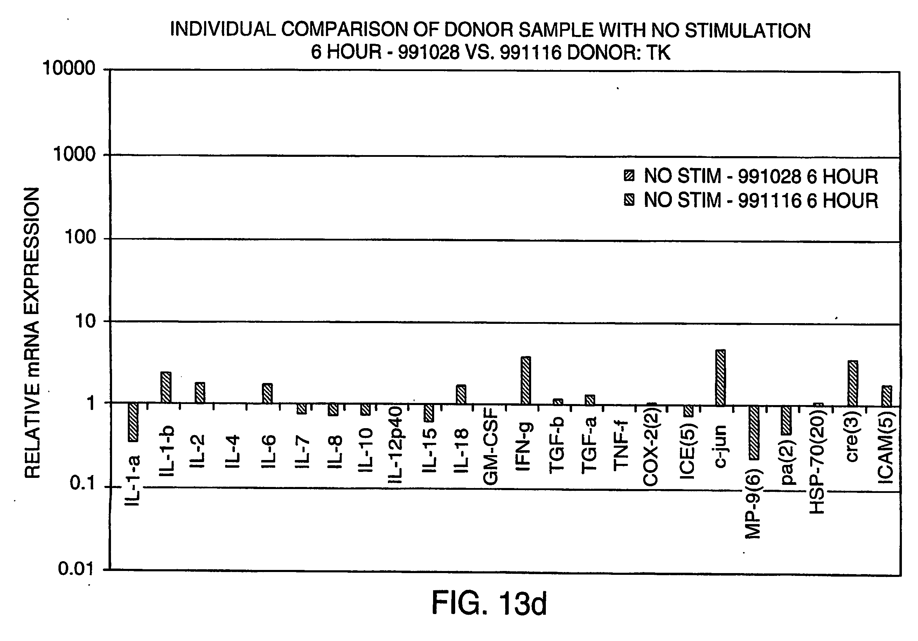

Figure 13 are bar graphs with a logarithmic axis that shows a graphical representation of calibrated profile data sets for two different samples of whole blood (a) 991116 and (b) 991028 reflecting the biological condition of the cells using a panel of 24 members, each member corresponding to a gene locus, the baseline profile data set being derived from untreated cells. The calibrated data sets for cells exposed for six hours to three inflammation inducing agents (lipopolysaccharide, heat killed staphylococci, and phytohemaglutinin) are compared for each sample. (c) shows a direct comparison of LPS stimulated 991116 with respect to 991028 as the baseline profile data set (d) shows a direct comparison between unstimulated 991116 and 991028.

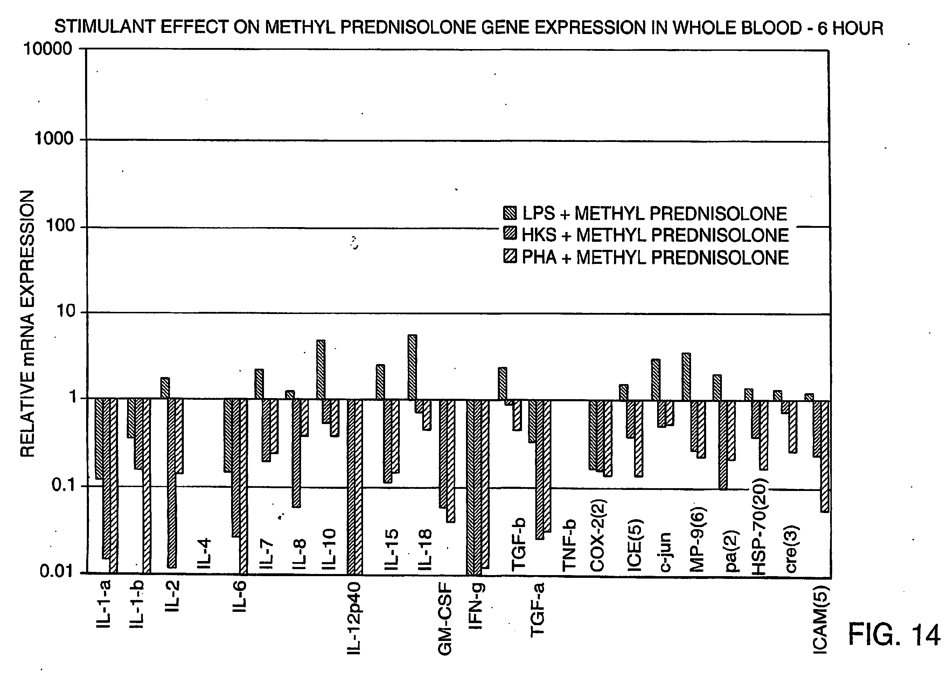

Figure 14 is a bar graph with a logarithmic axis that shows a graphical representation of calibrated profile data sets using a panel of 22 constituents, each constituent corresponding to a gene locus, the baseline profile data set being derived from untreated cells. Whole blood is exposed for six hours *ex vivo* to three inflammation inducing agents (lipopolysaccharide, heat killed staphylococci, and phytohemaglutinin) which are then treated with a single anti-inflammatory agent (methyl prednisolone) to reveal similarities and differences in the effect of a single agent on cell populations differing in their biological condition.

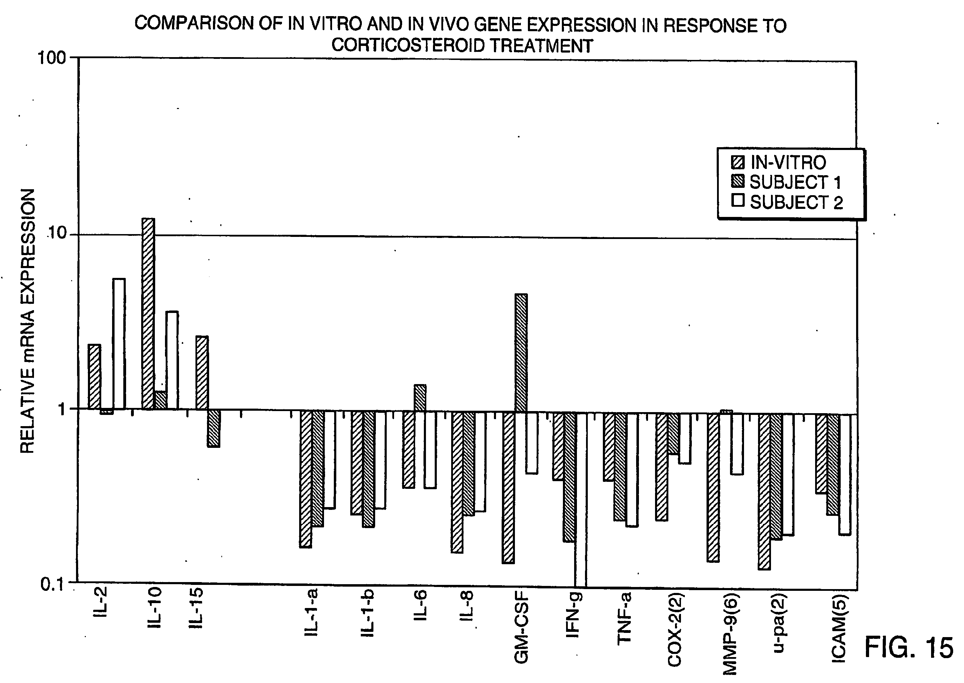

Figure 15 is a bar graph with a logarithmic axis that shows a graphical representation of calibrated profile data sets for whole blood where one calibrated data set refers to a subject (subject 2) who has been treated *in vivo* with a corticosteroid (dexamethasone), a second data set refers to the treatment of a blood sample from the same subject prior to *in vivo* treatment where that sample has been treated *ex vivo* (*in vitro*) and the third data set refers to a second subject treated *in vivo* with dexamethasone (subject 1). The data sets demonstrate the reproducibility and predictability of an *ex vivo* (*in vitro*) treatment of blood compared to *in vivo* treatment with the same agent. The figure also shows minor variation between samples from different subjects reflecting interpersonal variability. A panel of 14 constituents is provided. The baseline profile data set is derived from untreated whole blood from the cognate subject.

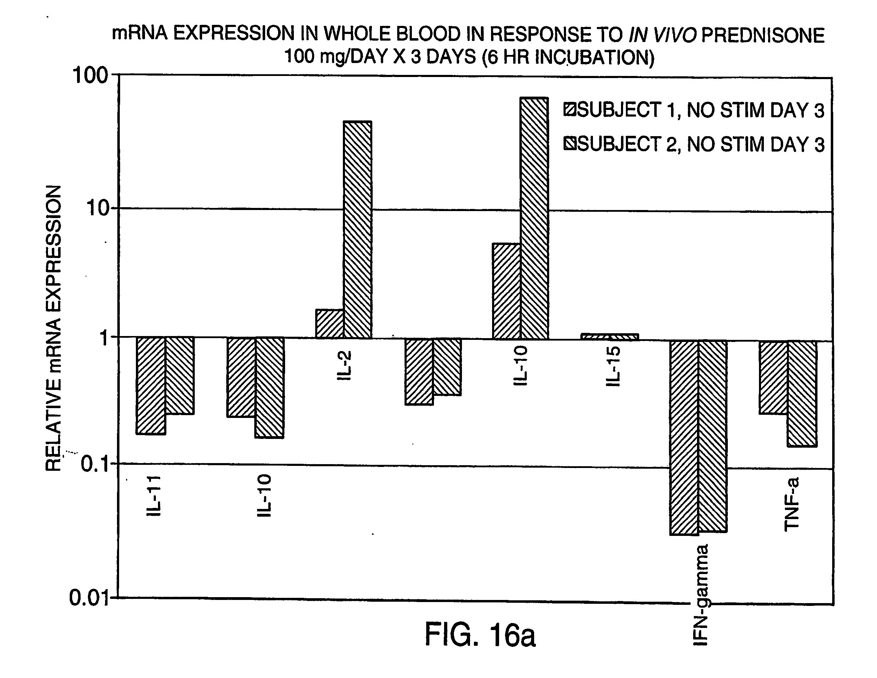

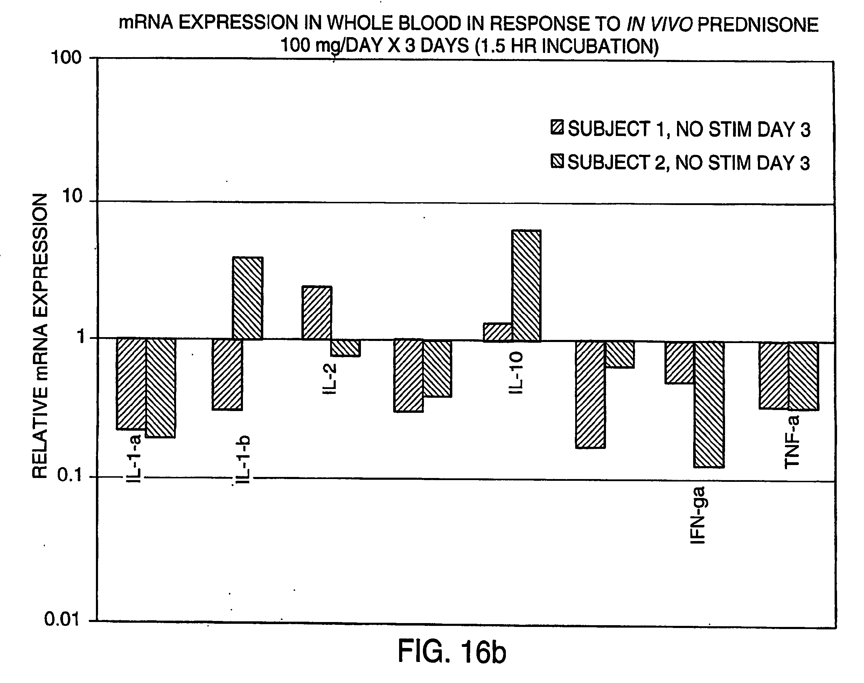

Figure 16 is a bar graph with a logarithmic y axis that shows a graphical representation of calibrated profile data sets for whole blood where one calibrated data set refers to (a) 2 subjects who have been treated *in vivo* with an inactive placebo for 3 days and (b) active prednisolone for 3 days at 100 mg/day. The data set shows some variation between samples from different subjects treated with the same drug. The data sets demonstrate similarity of responses across the same gene loci, as well as, quantitative variation at other loci suggesting quantifiable interpersonal variation. A panel of eight members is provided. The baseline profile data set is derived from untreated whole blood.

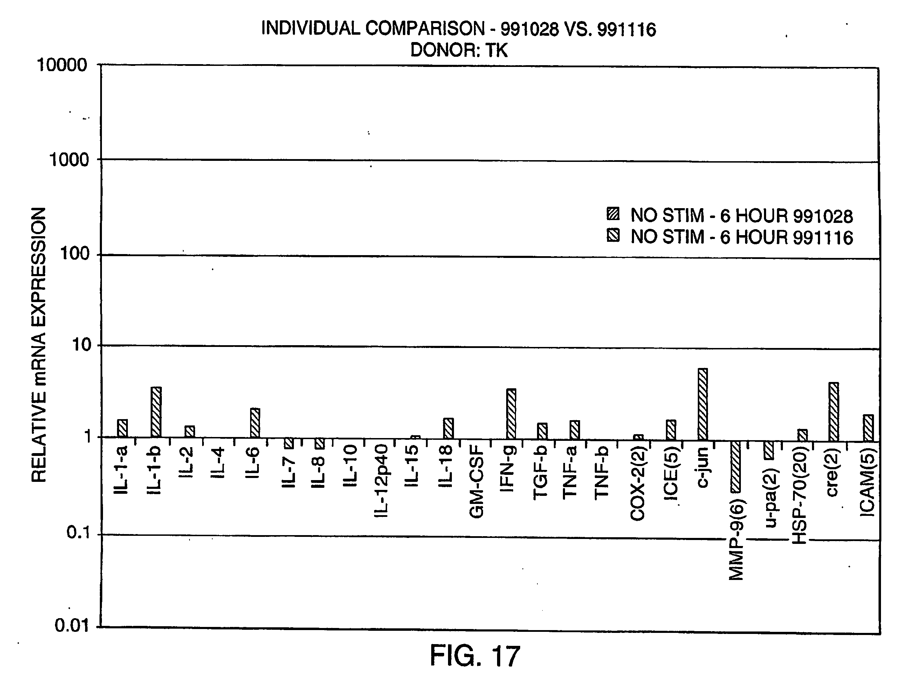

Figure 17 is a bar graph with logarithmic y axis that shows a graphical representation of calibrated precision profile data sets for two samples taken from a single subject within a 19 day period using a panel (e.g., inflammation panel) of 24 members where each member corresponds to a unique gene locus. The baseline profile data set relates to peripheral blood taken from the subject prior to treatment.

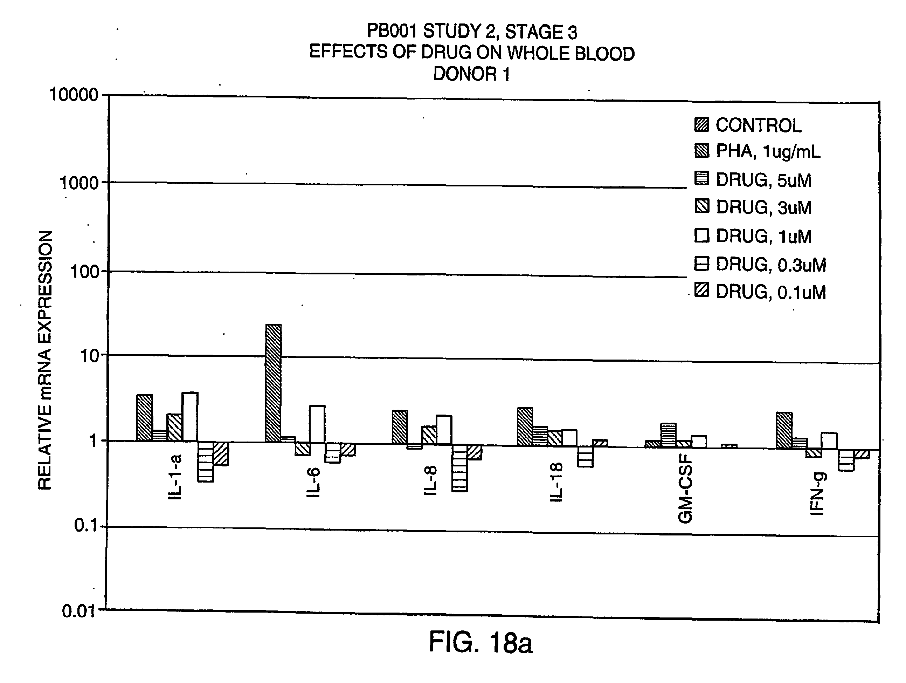

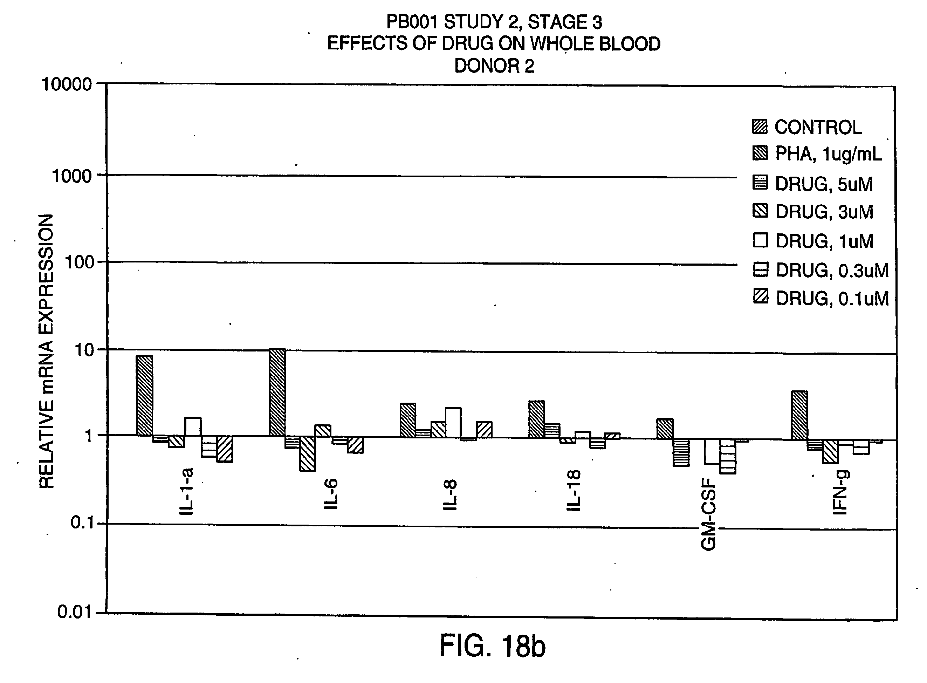

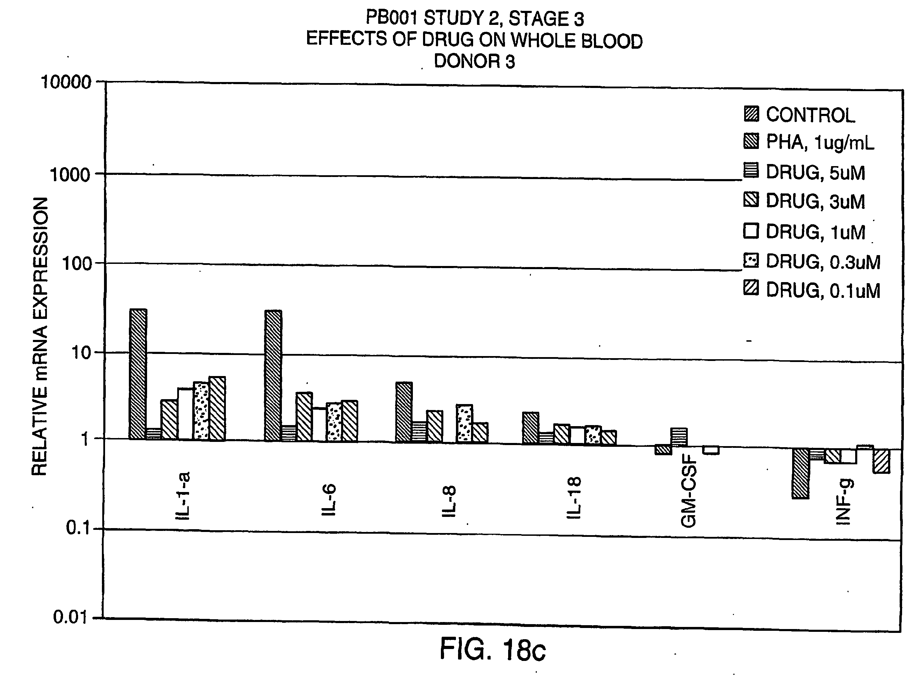

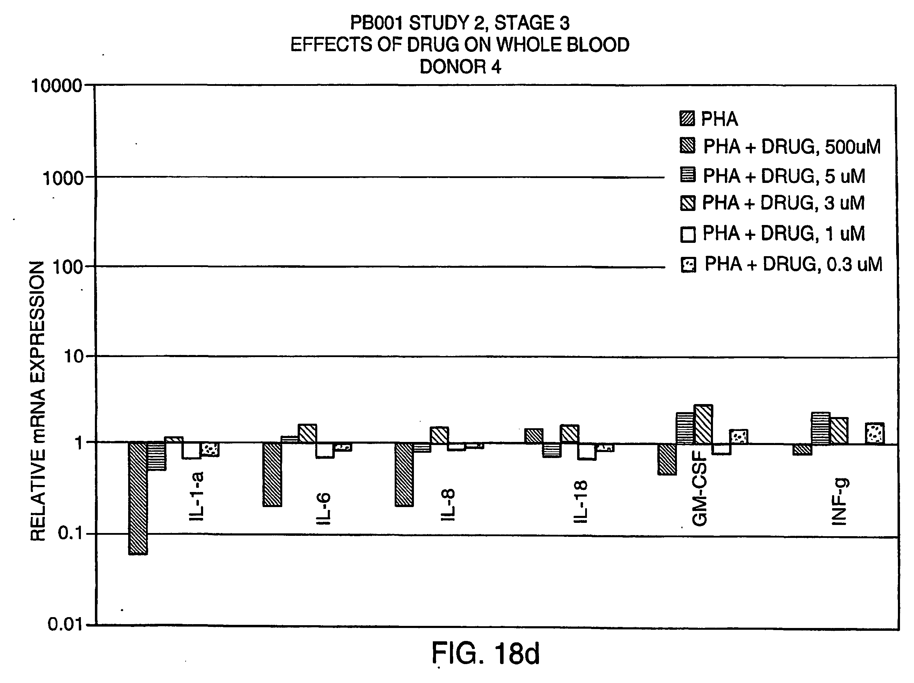

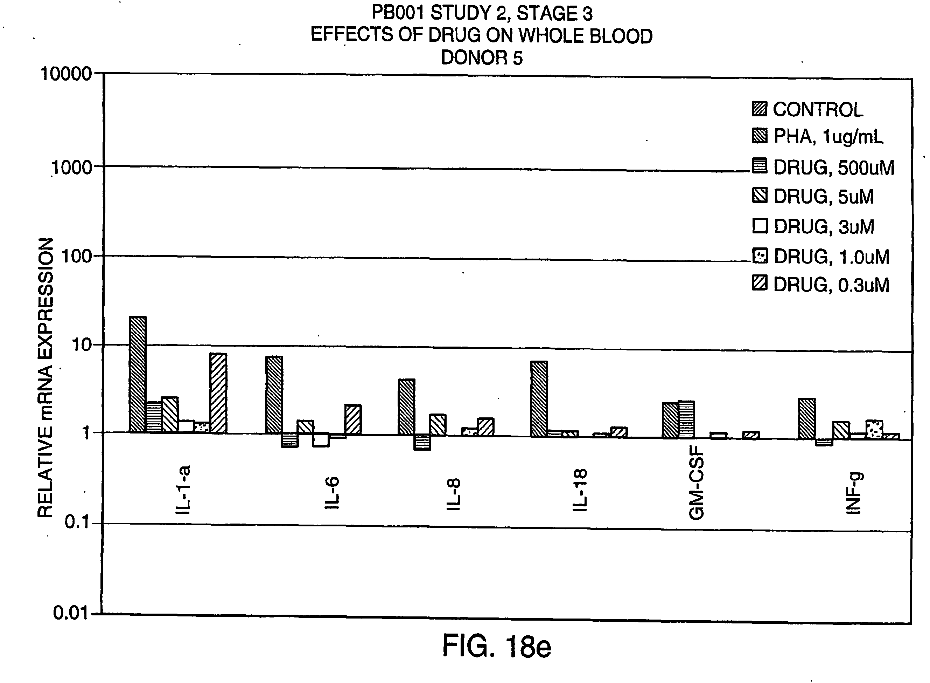

Figure 18 (a-e) are bar graphs with a logarithmic axis that show a graphical representation of calibrated profile data sets for each of 5 subjects from which a blood sample has been taken. Each of the blood samples was exposed to the inflammatory agent phytohemagglutin (PHA) or to a therapeutic agent (anti-inflammatory agent) at different concentrations: 0.1µM, 0.3uM, 1µM, 3µM and 5µM, for a 4 hour period *ex vivo* (*in vitro*) so as to determine the optimum dose for treating the subject. A panel of 6 constituents were used corresponding to 6 gene loci. The baseline profile data set was untreated sample obtained from the cognate donor.

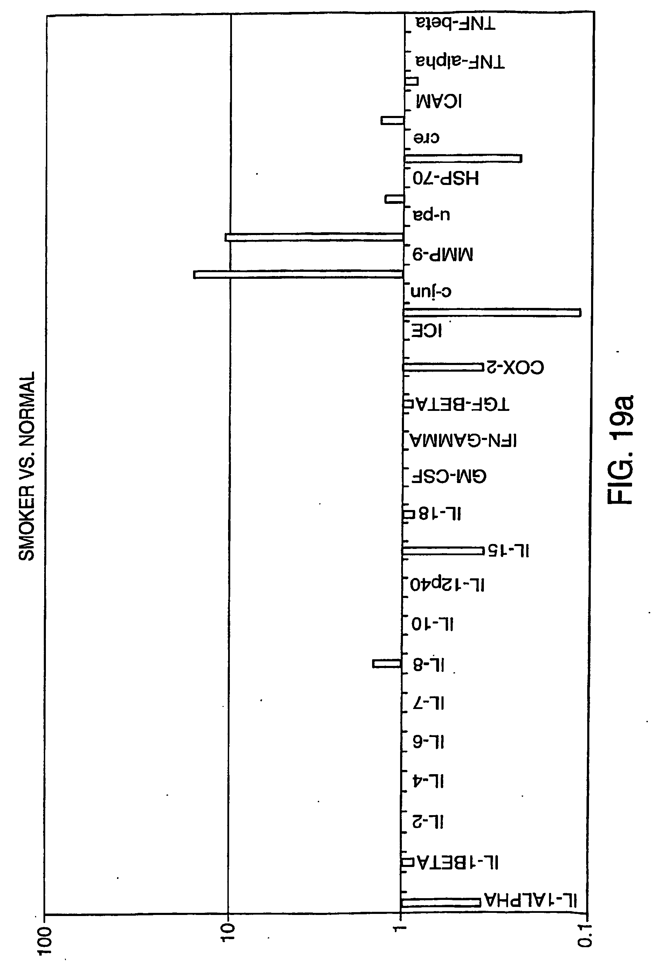

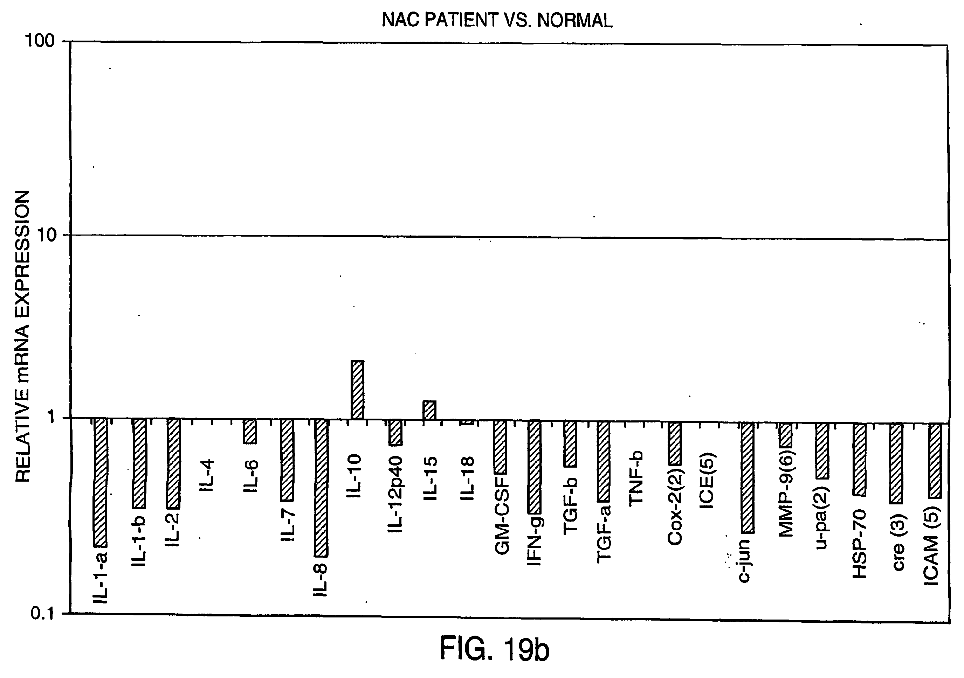

Figure 19 is a bar graph with a logarithmic axis that shows a graphical representation of calibrated profile data sets for three different subjects having different biological conditions using a panel with 24 constituents. The profile data sets show variability according to these conditions providing the basis for a diagnostic signature panel. (a) shows a calibrated profile data set for a smoker against a baseline for a non-smoker. (b) shows a calibrated profile data set for a subject with chronic obstructive pulmonary disease against a baseline for a subject lacking this disease. The baseline profile data set is derived from a subject that is "normal" with respect to these conditions.

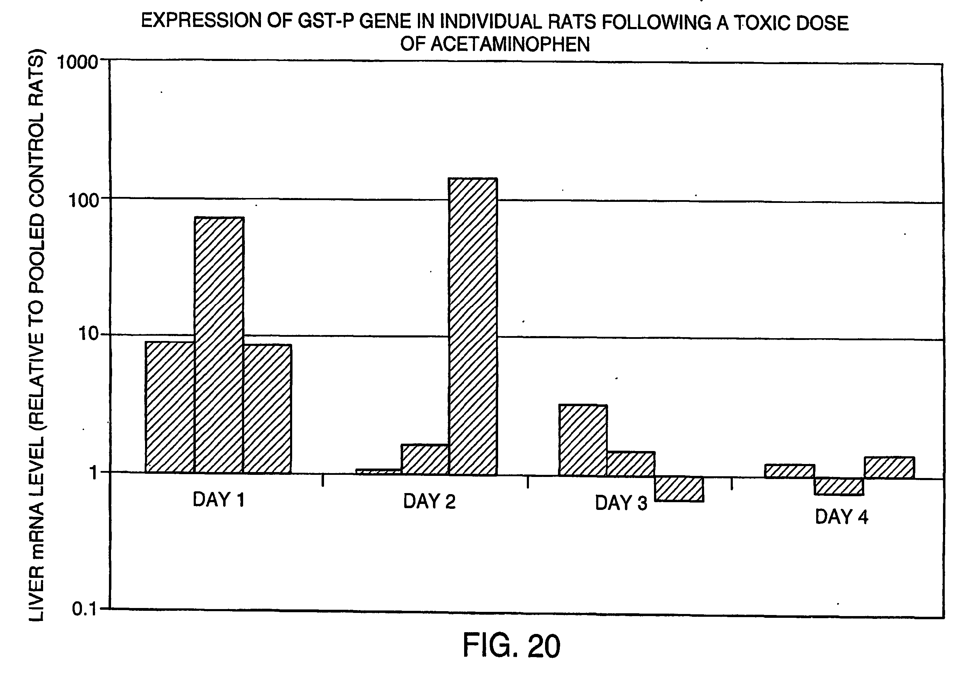

Figure 20 illustrates that an individual responses can be distinguished from a similarly treated population. A comparison of the response of a single animal compared to its experimental cohort (n=5 animals) with respect to a single locus (GST-P) is provided. The baseline data set is the cohort average. The figures shows that this animal varied significantly from the daily, population average in the first two days of the study, but became more similar to the cohort average with time after treatment with acetaminophen.

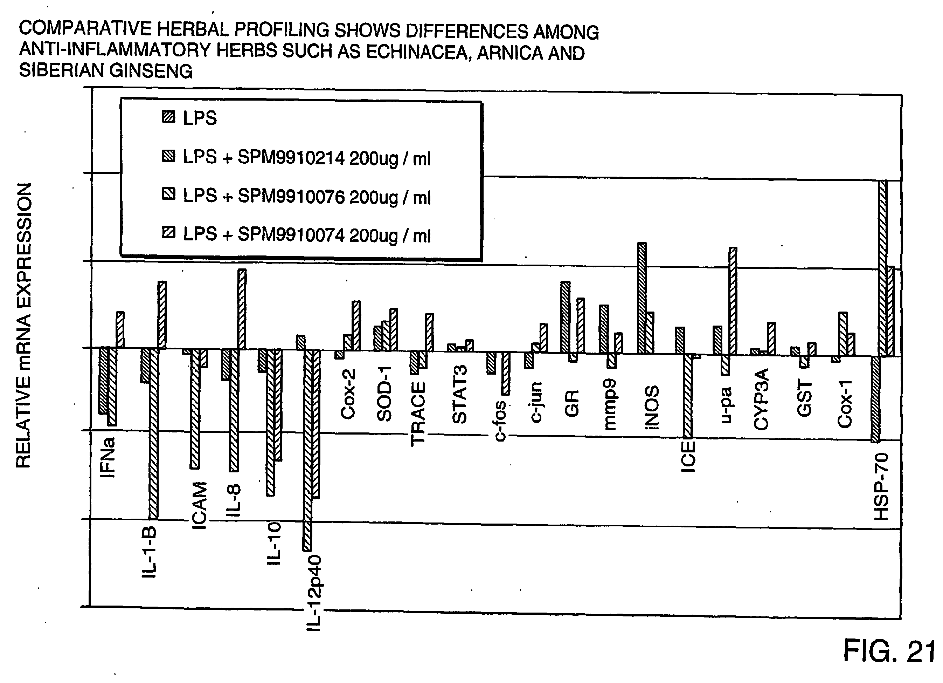

Figure 21 is a bar graph with a logarithmic axis that shows a graphical representation of calibrated profile data sets for samples of blood treated *ex vivo* with LPS or LPS and one of three anti-inflammatory herbals (Echinacea, Arnica or Siberian Ginseng) at a concentration of 200 ug/ml. A panel of 24 constituents is used. The baseline profile data set is derived from LPS stimulated cells absent a herbal treatment. The figure illustrates the effectiveness of the use of the calibrated precision profile to investigate the overall effects of complex compounds such as nutraceuticals whose biological effect is a summation of more than one activity. In this case, each of the herbals is consumed as an immunostimulant, however the calibrated precision profiles reveal a unique pattern shows a mixture of both immunostimulatory and anti-inflammatory effects.

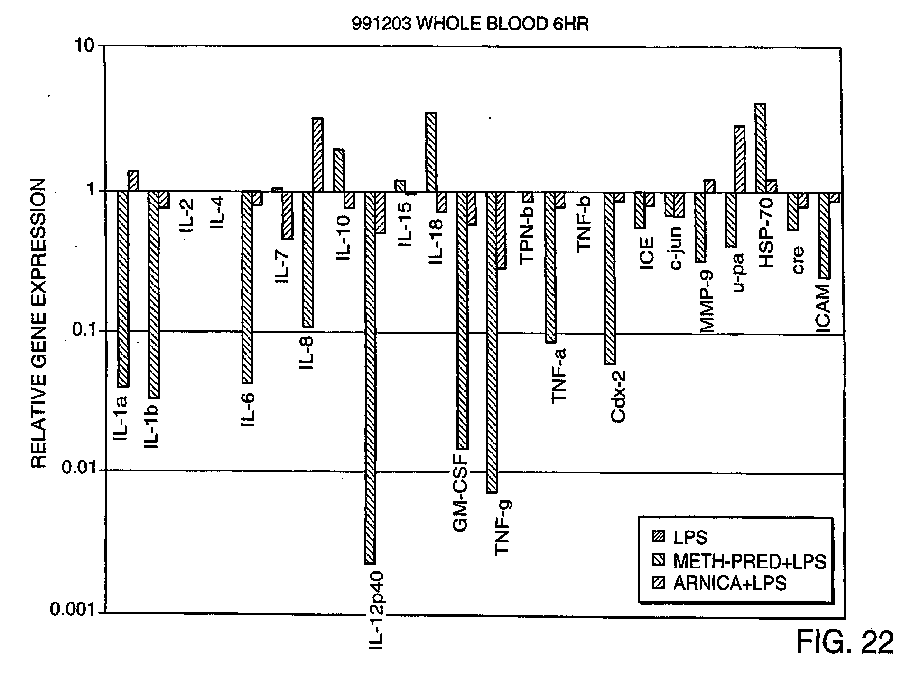

Figure 22 is a bar graph with a logarithmic axis that shows a graphical representation of calibrated profile data sets for samples of blood treated *ex vivo* with LPS or LPS and methylprednisolone or LPS and Arnica. The baseline profile data set is LPS treated blood sample.

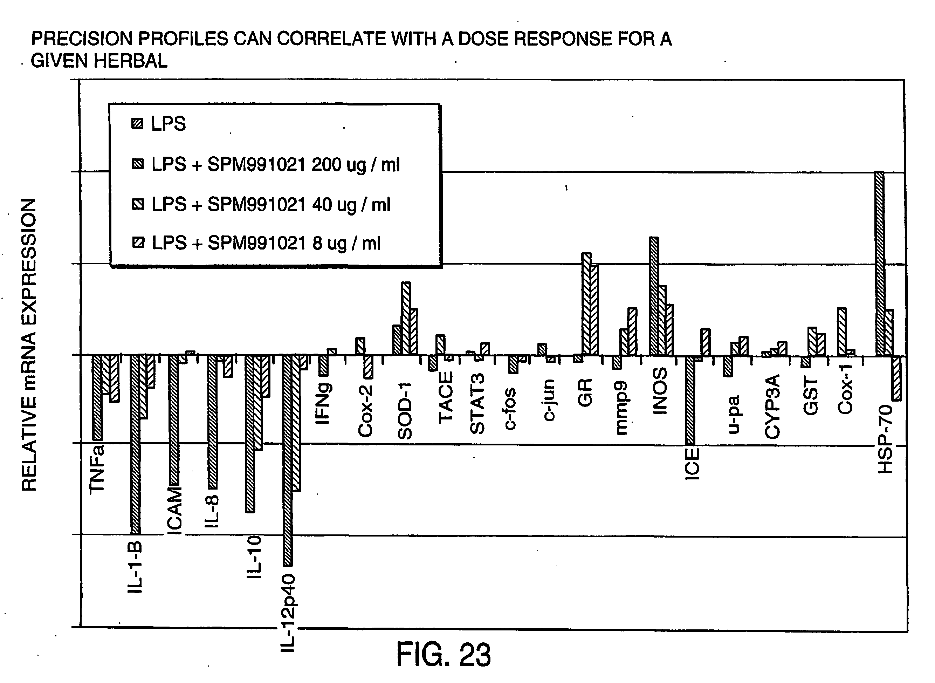

Figure 23 is a bar graph with a logarithmic axis that shows a graphical representation of calibrated profile data sets for samples of THP-1 cells treated with LPS or LPS and Arnica at three different concentrations using a panel of 22 constituents. The baseline profile data set is untreated THP-1 cells. The figure illustrates a concentration response with respect to the gene expression across the calibrated profile.

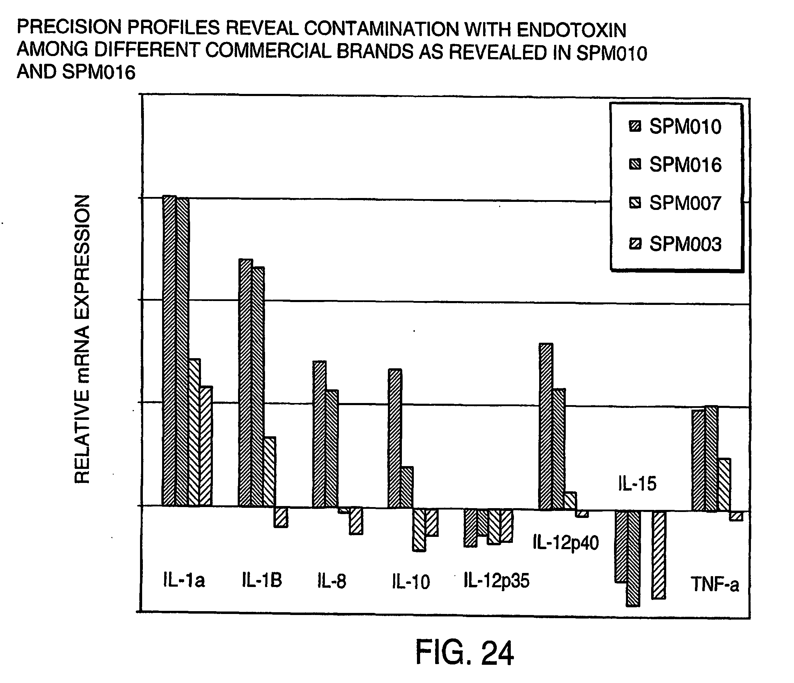

Figure 24 is a bar graph with a logarithmic axis that shows a graphical representation of calibrated profile data sets for samples of THP-1 cells treated *ex vivo* with four different commercial brands of Echinacea using a panel of 8 constituents. The baseline profile data set is untreated THP-1 cells.

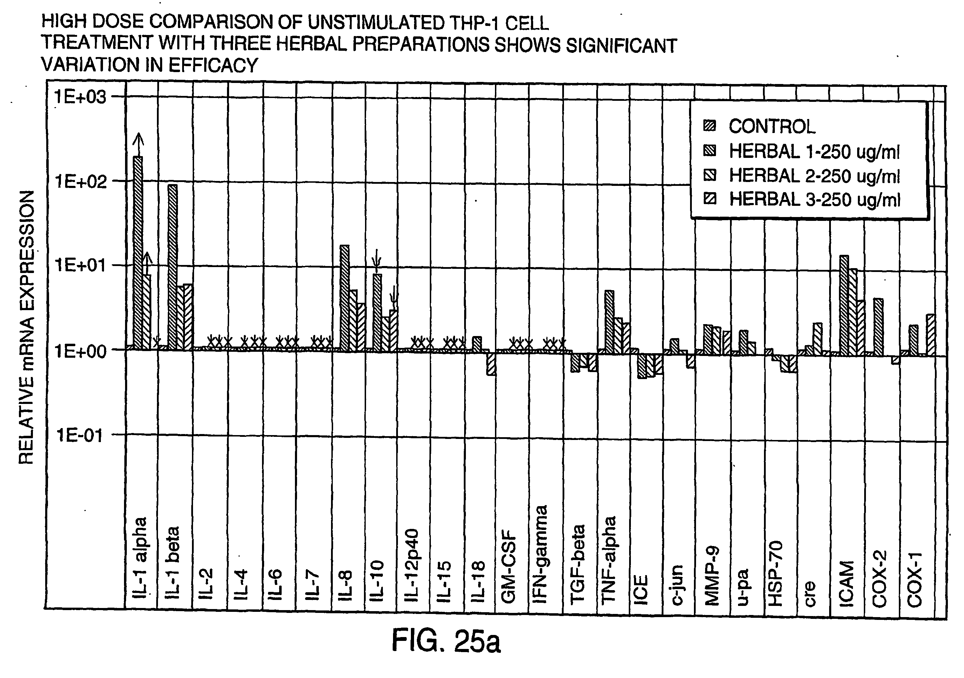

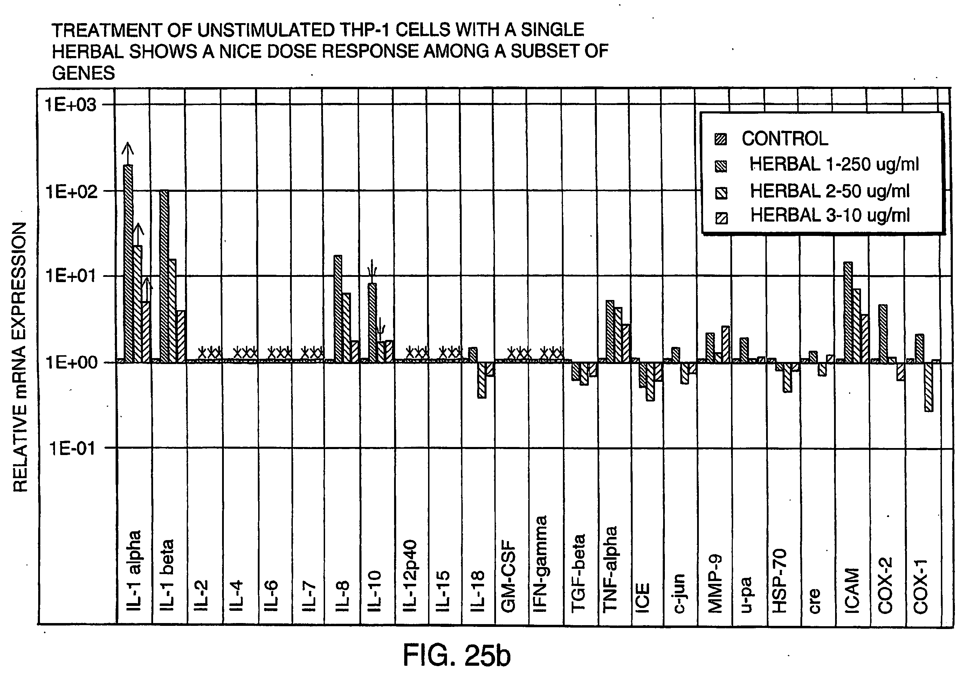

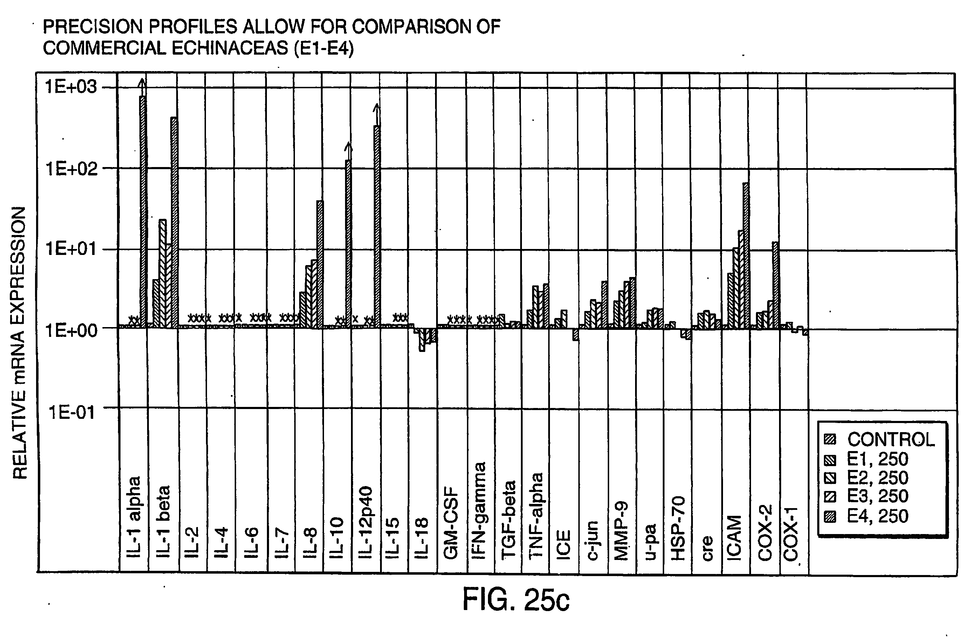

Figure 25 illustrates the use of the calibrated profile to compare relative efficacy across brands, or different formulations. Calibrated profile data sets for herbal preparations from different manufacturing sources with respect to an indicator monocytic cell line (THP-1) are shown graphically, the baseline profile data set being THP-1 cells absent the herbal. (a) three commercial herbal . Echinacea preparations at 250 (ug/ml); (b) three herbal preparations at different concentrations (250ug/ml, 50ug/ml and 3-10ug/ml) (c) four commercial Echinacea brands at 250 ug/ml).

### Detailed Description of Specific Embodiments

As used in this description and the accompanying claims, the following terms shall have the meanings indicated, unless the context otherwise requires:
A "collection of cells" is a set of cells, wherein the set has at least one constituent.
A "population of cells" includes one or more cells. A population of cells may refer to cells *in vivo* or to *in vitro* cultures. *In vitro* cultures may include organ cultures or cell cultures where cell cultures may be primary or continuous cell cultures of eukaryotic or prokaryotic cells. Cell lines can be primary cultures or cell samples, e.g. from a tumor, from blood or a blood fraction, or biopsy explants from an organ, or can be established cell lines or microbial strains.
A "region of the subject" from which proteins are obtained may be (but is not required to be) the same part of the subject from which has been obtained a collection of cells or a population of cells. The cells and the proteins may both be obtained from blood of the subject, for example. Alternatively, for example, the cells may be obtained from blood and the proteins may be obtained from a scraping of tissue or vice versa. Similarly, the proteins may be obtained from urine of the subject, for example, whereas the cells may be obtained elsewhere, as, for example, from blood.
A "panel" of genes is a set of genes including at least two constituents.
A "normative" condition of a subject to whom a composition is to be administered means the condition of a subject before administration, even if the subject happens to be suffering from a disease.
An "expression" of a gene includes the gene product whether messenger RNA or protein resulting from translation of the messenger RNA.
A "large number" of data sets based on a common panel of genes is a number of data sets sufficiently large to permit a statistically significant conclusion to be drawn with respect to an instance of a data set based on the same panel.
A *"*biological condition" of a subject is the condition of the subject in a pertinent realm that is under observation, and such realm may include any aspect of the subject capable of being monitored for change in condition, such as health, disease including cancer; trauma; aging; infection; tissue degeneration; developmental steps; physical fitness; obesity, or mood. As can be seen, the conditions may be chronic or acute or simply transient. Moreover, a targeted biological condition may be manifest throughout the organism or population of cells or may be restricted to a specific organ (such as skin, heart, eye or blood). The term "biological condition" includes a "physiological condition".
The "blind administration" of a selected one of a composition or placebo to a subject in a clinical trial involves administering the composition or placebo to the subject in accordance with a protocol pursuant to which the subject lacks knowledge whether the substance administered is the composition or a placebo.
An "organism" is any living cell including microorganisms, animals and plants. An animal is commonly in this context a mammal, but may be a vertebrate non-mammal, as e.g., a zebra fish, or an invertebrate, as, e.g.

### Caenorhabditis elegans.

An "agent" is a composition or a stimulus. A "stimulus" may include, for example ultraviolet A or B, or light therapy for seasonal affective disorder, or treatment of psoriasis with psoralen or treatment of melanoma with embedded radioactive seeds, other radiation exposure, etc. A "composition" includes a chemical compound, a nutraceutical, a combination of compounds, or a complex mixture.

A "clinical indicator" is any physiological datum used alone or in conjunction with other data in evaluating the physiological condition of a collection of cells or of an organism. This term includes pre-clinical indicators.

A "signature panel*"* is any panel representing a subclass of constituents where the subclass of constituents is selected according to the relatively high level of information concerning a biological condition imparted by each member of the data set.

"Distinct RNA or protein constituent" in a panel of constituents is a panel that includes at least one of RNA and protein and each constituent of the panel is distinct.

The present invention comprehends the formation of calibrated data sets that describe a biological condition or an effect of an agent on a biological condition. A calibrated data set represents a set of values that correspond to variations in gene expression where the variations are informative. This approach does not require comprehensive analysis of all gene expression in target cells associated with a particular condition. Nor is any one single gene locus necessarily of particular significance. Rather a pattern of variation (a profile) is sought that correlates, in a reproducible manner, with a particular condition. There may be no *a priori* knowledge of a correlation but rather a correlation may be established by evaluating a panel of constituents of reasonable size (for example up to 100 constituents) and iteratively testing the gene expression profiles for different subjects or for the same subject from which the most informative loci for a particular condition may be selected. An informative subgroup of constituents in a panel may be selected that consistently vary for a particular condition and this subgroup may then become the signature panel, the signature panel giving rise to a signature profile.

In further embodiments of the invention, any calibrated data set for an individual that has more members than reflective of a single signature panel may be mined for calibrated profiles that correspond to additional signature panels thereby potentially providing new insights into mechanisms of action of a biological condition on sets of genes. Measurement of changes in transcribed RNA in a cell as a result of an environmental change or aging is an exquisitely sensitive measure of the response of a cell. Techniques available today to quantify transcribed RNA in a cell add to the sensitivity of the approach. The preferred embodiments of the invention, which are directed to patterns of change in amounts of transcribed RNA, provide a means to focus and interpret this rich information.

In contrast to the above approach, much attention in the prior art has been directed to the sequencing of the human genome and the identification of all the genes encoded therein. Accompanying the growing amount of sequence data, microarrays provide a means to survey thousands of gene sequences for mutations. Microarrays are being used to provide DNA profiles that identify mutations in an individual and those mutations will be associated with predictions concerning development of disease in those individuals. Transcriptomics and proteomics is now the focus of increasing attention. These studies are directed to analyzing the entire body of RNA and protein produced by living cells. Microarrays provide a method for analyzing many thousands of different human RNAs as to whether they are expressed and by which cells. For example, a project undertaken by the National Cancer Institute and others to examine mRNAs produced by various types of cancer cells, have revealed 50,000 genes that are active in one or more cancers. The goal of these studies is to identify novel cancer drugs that are directed to knocking out or enhancing the production of certain proteins. (Kathryn Brown, The Human Genome Business Today, Scientific American, July 2000, p.50; Julia Karow, The "Other" Genomes, Scientific American, July 2000, p.53; Ken Howard, "The Bioinformatics Gold Rush, Scientific American, July 2000, p.58; Carol Ezzell, Beyond the Human Genome, Scientific American, July 2000, p.64; all incorporated by reference). Major efforts in correlating genetic variation of individuals and the functional interrelationships of genes in health and disease are being conducted in a variety of consortia including the single nucleotide polymorphism consortium and the Human Epigenome Consortium (Beck et al. Nature BioTechnology 17 (1999) p 1144). The Epigenome Consortium plans to analyze sets of genome fragments from both healthy and diseased individuals in the 500 different human tissues (Bioworld International: December 22,1999). This approach seeks to correlate absolute expression of genes associated with a particular condition with the presence of that condition. Examples of prior art that seek to measure gene expression in absolute amounts including by subtractive methods or by determining amounts with respect to housekeeping genes or by targeting a single gene expression system are U.S. 5,643,765; U.S. 5,811,231; U.S. 5,846,720; U.S. 5,866,330; U.S. 5,968,784; U.S. 5,994,076; WO 97/41261; WO 98/24935; WO 99/11822; WO 99/44063; WO 99/46403; WO 99/57130; WO00/22172 and WO00/11208.

We have taken a different and novel approach to the above by identifying reproducible patterns of variation of gene expression that are informative by virtue of the degree of variation between a sample and a baseline for example a subject with the condition and the subject that lacks the condition. The variations may be correlated with other non- genetic indications such as clinical indicators (for humans) of a traditional nature but are not required per se to be causative. Accordingly, the amount of gene expression product (for example RNA transcript) produced by a gene locus in a cell under certain circumstances is measured and then stored as a value in a first profile data set. This value is calibrated with respect to a second value (a baseline profile data set) to provide a member of a calibrated profile data set. The values recorded for the profile data set, relying on a particular baseline data set to produce a calibrated data set become part of the descriptive record any or all of which can be stored in a database which may be accessed through a global network such that any new data in the form of a profile data set or a calibrated profile data set measured at any global location can be directly compared to an archive of descriptive records including calibrated profile data sets and baseline data sets so as to extend the stored library of profiles and provide predictive or diagnostic data about a particular biological condition or agent.

We have exemplified the use of selected panels of constituents corresponding to gene loci from which quantitative gene expression is measure by for example quantitatively measuring the transcribed RNA in a sample of a subject, for applications that include: (a) measurement of therapeutic efficacy of natural or synthetic compositions or stimuli that may be formulated individually or in combinations or mixtures for a range of targeted physiological conditions; (b) predictions of toxicological effects and dose effectiveness of a composition or mixture of compositions for an individual or in a population; (c) determining how two different agents administered in a single treatment might interact so as to detect any of synergistic, additive, negative, neutral of toxic activity (d) performing pre-clinical and clinical trials by providing new criteria for pre-selecting subjects according to informative profile data sets for revealing disease status and conducting preliminary dosage studies for these patients prior to conducting phase 1 or 2 trials. Gene expression profiling may be used to reduce the cost of phase 3 clinical trials and may be used beyond phase 3 trials; (e) labeling for approved drugs; (f) selection of suitable medication in a class of medications for a particular patient that is directed to their unique physiology; (g) diagnosing or determining a prognosis of a medical condition or an infection which may precede onset of symptoms or alternatively diagnosing adverse side effects associated with administration of a therapeutic agent; (h) managing the health care of a patient; and (i) quality control for different batches of an agent or a mixture of agents.

### The Subject

The methods herein can be applied to a subject that includes any living organism where a living organism includes a prokaryote such a bacterium or a eukaryote including single celled eukaryotic organisms at one end of the spectrum and humans at the other and everything in between including plants. The figures relate to calibrated profile data sets obtained from humans and mammals. Nonetheless, the methods disclosed here may be applied to cells of other organism without the need for undue experimentation by one of ordinary skill in the art because all cells transcribe RNA and it is know in the art how to extract RNA from all types of cells.

A tissue sample might include a single cell or multiple cells or fragments of cells. Body fluid includes blood, urine, spinal fluid, lymph, mucosal secretions, hemolymph or any other body fluid known in the art for a subject. For an animal subject, a tissue or fluid sample may be obtained by means of a biopsy needle aspirate, a lavage sample, scrapings and surgical incisions or other means known in the art.

### Panels

Steps in selecting constituents in a panel include searching publicly available medical literature for RNA or proteins or sets of RNAs or proteins that directly or indirectly vary with a particular biological condition. A panel containing up to 100 constituents may be selected. According to the condition being examined, just a small subset of the panel constituents may be informative. In determining membership of the panel of genes, it is not necessary for the panel to be an exhaustive selection. Rather it is desired to obtain from the panel an expression profile that discriminates consistently with respect to the targeted physiological or biological condition. Moreover, a panel is not necessarily selected according to an expected profile of gene expression in cells that directly respond to a biological effect. For example, gene expression associated with liver metabolism may be analyzed in a blood sample. Figures 20 and 22 provide calibrated profiles of whole blood treated with herbal agents using markers for liver metabolism.

The number of constituents in a panel can vary. According to the examples provided below, panels of up to 24 genes are selected for evaluating expression levels. Although a panel may be as large as 100 constituents, it is desirable for a particular panel to have no more than 24 constituents, more particularly, less than 12 constituents. For example, subsets of no more than 8 genes have been used that may be derived from a larger panel but which are sufficiently informative to effectuate discrimination. The number of constituents in a panel for which expression is monitored may vary widely depending on the context. For example, Figure 1 describes data acquisition from *in vitro* cell culture and from animal toxicology studies, which includes expression of about 25 to 100 or more genes. In contrast, selection of markers or surrogate markers include for example three to 100 genes, preferably five to 50 or five to 25 genes to be analyzed from samples obtained in clinical studies. In this manner markers or surrogate markers having predictive value for a medical condition, such as a genetic predisposition, a response to therapeutic agent, an inflammatory condition, or an infection, etc. can be identified and cumulatively larger populations can be obtained to refine the correlations. A health profile can then be generated for an individual subject using a low volume blood sample. The blood sample can be analyzed for expression profile data of about 100 - 500 genes, comprising markers or surrogate markers of a number of medical conditions (Fig. 1: right panel). Panels of varying sizes may be utilized as necessary and subsequent refinements in methodology may lead to selection of subsets having panels as large as 15 genes or 12 genes or as small as 6, 5, 4, 3 genes.

It is envisaged that any single biological condition may be described by a signature panel having a small number of highly informative constituents providing a signature calibrated profile (also referred to as a fingerprint). The presence of highly informative loci is demonstrated in several of the accompanying figures. For example, Figure 11(a) Il-2, Il-4 and Il-5 appeared to be highly informative. Highly informative constituents in Figure 21 include the interleukins. The signature panel may provide a signature profile or fingerprint which is sufficiently robust to serve as a standard in describing a particular biological condition or an effect of a particular agent on a biological condition.

For purposes of illustrating a signature panel, constituents of a panel for measuring inflammation have been provided that are informative with respect to a particular biological condition. For example, we have used a panel for inflammation that has 6 constituents- Il-1a, Il-6, Il-8, Il-18, GMCSF and IFN-g in Figure 18(a)-(e) to determine the response of 5 subjects to varying concentrations of drugs. This group of constituents is a subset of a larger panel of inflammation related gene loci such as shown in Figure 19a and Figure 19b where the Inflammatory Panel includes Il-a, Il-b, Il-2, Il-3, Il-4, Il-6, Il-7, Il-8, Il-10, Il-12p40, Il-15, Il-15, Il-18, GM-CSF, Ifn-gamma, TGF-b, cox-2, ICE, MMP-9, ICAM, TNF-a and TNF-b. The subset of constituents were selected on the basis of the information sought concerning the biological condition.

Embodiments of the invention provide examples of at least 4 different panels which may be used separately or together. These panels are an inflammatory panel (TNF-a, II-1b, ICAM, II-8, II-10, II-12p40, ICE, cox-2, cox-1 and mmp-3) a cell growth and differentiation panel (c-fos, c-jun and STAT3), a toxicity panel (SOD-1, TACE, GR, HSP70, GST, c-fos, c-jun, INOS) and a liver metabolism panel (INOS, cyp-a and u-pa). Other panels include skin response or prostate cancer or endothelial/cardiovascular response panels or cell growth or differentiation or liver metabolism panels. Although provided as examples, the above panels are not intended to be limiting.

### Gene Expression

For measuring the amount of a particular RNA in a sample, transcribed RNA from a sample is extracted and quantified with respect to a constituent of a panel. RNA is extracted from a sample such as a tissue, body fluid, or culture medium in which a population of a subject might be growing. For example, cells may be lysed and RNA eluted in a suitable solution in which to conduct a DNAse reaction. First strand synthesis may then performed using a reverse transcriptase. Gene amplification, more specifically quantitative PCR assays, can then conducted and the gene of interest size calibrated against a marker such as 18S rRNA (Hirayama et al., Blood 92,1998:46-52). Samples are measured in multiple duplicates for example, 4 replicates. Relative quantitation of the mRNA is determined by the difference in threshhold cycles between the size marker and the gene of interest. In an embodiment of the invention, quantitative PCR is performed using amplification, reporting agents and instruments such as those supplied commercially by PE Biosystems (Foster City, CA). Given a defined efficiency of amplification of target transcripts, the point (e.g., cycle number) that signal from amplified target template is detectable may be directly related to the amount of specific message transcript in the measured sample. Similarly, other quantifiable signals such as fluorescence, enzyme activity, disintegrations per minute, absorbance, etc., when correlated to a known concentration of target templates (e.g., a reference standard curve) or normalized to a standard with limited variability can be used to quantify the number of target templates in an unknown sample.

Although not limited to amplification methods, quantitative gene expression techniques may utilize amplification of the target transcript. Amplification of the target template may be accomplished by isothermic gene amplification strategies, or by gene amplification by thermal cycling such as PCR. It is desirable to obtain a definable and reproducible correlation between the amplified target or reporter and the concentration of starting templates.

It is envisaged that techniques in the art using microfluidics for example and highly sensitive markers will enable quantitation of RNA to occur directly from a single cell or lysed cell. The amount of transcript measured for any particular locus is a data point or member of the first profile data set for a particular panel.

According to embodiments of the invention, a first profile data set is derived from the sample, the first profile data set including a plurality of members, each member being a quantitative measure of the amount of a RNA transcribed from a gene locus, the gene locus being a constituent in a panel of constituents. A first profile data set may be obtained from a quantitative measure of the amount of a distinct RNA or protein corresponding to a gene locus. The figures provided here are directed to RNA. However, the method could be applied using proteins where sensitive quantitative techniques are available for measuring the amount of a distinct protein in a cell.

### Baseline Profile Data Sets

The analyses of samples from single individuals and from large groups of individuals provide a library of profile data sets relating to a particular panel or series of panels. These profile data sets may be stored as records in a library for use as baseline profile data sets. As the term "baseline" suggests, the stored baseline profile data sets serve as comparators for providing a calibrated profile data set that is informative about a biological condition or agent. It is anticipated that many baseline profile data sets will be stored in libraries and classified in a number of cross-referential ways. One form of classification might rely on the characteristics of the panels from which the data sets are derived. Another form of classification might be the use of a particular biological condition. The concept of biological condition encompasses any state in which a cell or population of cells might be at any one time. This state might reflect geography of samples, sex of subjects or any other discriminator. Some of the discriminators may overlap. The libraries might also be accessed for records associated with a single subject or particular clinical trial. The classification of baseline profile data sets may further be annotated with medical information about a particular subject, a medical condition, a particular agent etc.

The choice of a baseline profile data set for creating a calibrated profile data set is related to the biological condition to be evaluated, monitored, or predicted, as well as, the intended use of the calibrated panel, e.g., as to monitor drug development, quality control or other uses. It might be desirable to access baseline profile data sets from the same subject for whom a first profile data set is obtained or from different subject at varying times, exposures to stimuli, drugs or complex compounds; or may be derived from like or dissimilar populations.

The profile data set may arise from the same subject for which the first data set is obtained, where the sample is taken at a separate or similar time, a different or similar site or in a different or similar physiological condition. For example, Figure 5 provides a protocol in which the sample is taken before stimulation or after stimulation. The profile data set obtained from the unstimulated sample may serve as a baseline profile data set for the sample taken after stimulation. The baseline data set may also be derived from a library containing profile data sets of a population of subjects having some defining characteristic or biological condition. The baseline profile data set may also correspond to some *ex vivo* or *in vitro* properties associated with an *in vitro* cell culture. The resultant calibrated profile data sets may then be stored as a record in a database or library (Figure 6) along with or separate from the baseline profile database and optionally the first profile data set although the first profile data set would normally become incorporated into a baseline profile data set under suitable classification criteria.

Selected baseline profile data sets may be also be used as a standard by which to judge manufacturing lots in terms of efficacy, toxicity, etc. Where the effect of a therapeutic agent is being measured, the baseline data set might correspond to gene expression profiles taken before administration of the agent. Where quality control for a newly manufactured product is being determined, the baseline data set might correspond with a gold standard for that product. However, any suitable normalization techniques may be employed. For example, an average baseline profile data set is obtained from authentic material of a naturally grown herbal nutraceutical and compared over time and over different lots in order to demonstrate consistency, or lack of consistency, in lots of compounds prepared for release.

### Calibrated Data

A calibrated profile data set may be described as a function of a member of a first profile data set and a corresponding member of a baseline profile data set for a given gene locus in a panel. For example, calibrated profile data sets may be derived by calculating a ratio of the amount of RNA transcribed for a panel constituent in a cell sample in an environmental including intervention such as a therapeutic treatment or at a particular time (first profile data set) with respect to the amount of RNA transcribed for the same panel constituent in a cell that differs in some manner from the sample (baseline profile data set) (Figures 5 and 6). We have found that calibrated profile data sets to be reproducible in samples that are repeatedly tested (Figure 17). We have also found that calibrated profile data sets obtained when samples from a subject are exposed *ex vivo* to a compound are comparable to calibrated profile data from a sample that has been exposed to a sample *in vivo* (Figure 14 and Figure 16(a),(b)). We have also found that an indicator cell line treated with an agent can provide comparable calibrated profile data sets to those obtained from *in vivo* or *ex vivo* populations of cells (Figure 15). Moreover, we have found that administering a sample from a subject onto indicator cells can provide informative calibrated profile data sets with respect to the biological condition of the subject including the health, disease states, therapeutic interventions, aging or exposure to environmental stimuli or toxins of the subject (Figure 25).

A preferred use of a calibrated profile data set is to evaluate a biological condition of a subject. This may be for purposes of diagnosis or prognosis of a clinical disorder. It is desirable to obtain a calibrated data set that describes a state of health or alternatively a state of age or body mass or any condition or state that an individual subject might find themselves to be in. For example, the biological condition might relate to physical activity, conditioning or exercise, mental state, environmental factor such as medication, diet, or geography or exposure to radiation or environmental contamination or infectious agent, biological or environmental toxin. If health or conversely a clinical disorder is being evaluated, calibrated profiles data sets may be used for monitoring change in health status by periodic or regular comparison of profiles; the disorder may be a complex disease process possibly involving multiple gene including inflammation, autoimmune disease, degenerative disease, allergy, vascular disease, ischemia, developmental disease, hormonal conditions and infectious diseases. The clinical disorder may further include arthritis, asthma, multiple sclerosis and perimenopausal changes. The biological condition may affect a system of a subject including a respiratory, vascular, nervous, metabolic, urinary, reproductive, structural and immunological system or other metabolic state. The above examples of a biological condition are given by way of illustration and are not intended to be limiting.

Similarly, calibrated profile data sets can be used to measure, monitor or predict the host response to an infectious agent for purposes of identifying the infectious agent, assessing the duration of infection, the extent of exposure or making therapeutic decisions.

The evaluation of activity of an agent may require a series of calibrated profiles. It is here shown that calibrated profile data sets can be used to describe the biological activity of an agent that might be a single compound or a complex compound such as a nutraceutical or herbal. The agent can be assayed using indicator cells, *ex vivo* cell populations or by *in vivo* administration. These assays may rely on a series of signature panels or enlarged panels for different biological conditions. The resultant calibrated profiles may then be used to infer likely *in vivo* activity from the *in vitro* study. Insights into toxicity and mechanisms of action can also be inferred from calibration profile data sets. For example, the herbal Echinacea is believed to have both immunostimulatory and anti-inflammatory properties although neither has been measured systemmatically. We have provided a systematic approach to investigate the biological activities of these and other herbs. We investigated the alleged immunostimulatory properties of the herbs by comparing the effect of treating the indicator cell line THP-1 or peripheral blood cells with the agent to untreated cells. Untreated cells include LPS stimulated untreated cells. Untreated cells were used as a baseline profile data set to measure the difference in gene expression between a baseline profile data set and the experimental treatment with the compound. Baseline profile data sets included a single sample or an average value from a series of experiments. The resultant calibrated profile data sets could then be compared with a library of calibrated profile data sets for a particular herb or/and libraries associated with different agents or conditions.

From the information obtained about a previously undescribed agent, a signature panel may be derived optionally together with a signature profile to serve as a gold standard for testing other batches of the same agent.

### Calculation of Calibrated Profile Data Sets and Computational Aids

The function relating the baseline and profile data sets is in a preferred embodiment, a ratio expressed as a logarithm. The calibrated profile data set may be expressed in a spreadsheet or represented graphically for example, in a bar chart or tabular form but may also be expressed in a three dimensional representation. Preferably the constituent is itemized on the x-axis and the logarithmic scale is on the y-axis. Members of a calibrated data set may be expressed as a positive value representing a relative enhancement of gene expression or as a negative value representing a relative reduction in gene expression with respect to the baseline.

Each member of the calibrated profile data set should be reproducible within a range with respect to similar samples taken from the subject under similar conditions. For example, the calibrated profile data sets may be reproducible within one order of magnitude with respect to similar samples taken from the subject under similar conditions. More particularly, the members may be reproducible within 50% more particularly reproducible within 20%. Each member of the calibrated profile data set has a biological significance if it has a value differing by more than an amount D, where D=F(1.1)-F(.9) and F is a second function.

It is the pattern of increasing, decreasing and no change in gene expression from the plurality of gene loci examined in the panel that is used to prepare a calibrated profile set that is informative with regards to a biological condition, biological efficacy of an agent treatment conditions or for comparison to populations and which may be used to identify likely candidates for a drug trial, used in combination with other clinical indicators to be diagnostic or prognostic with respect to a biological condition or may be used to guide the development of a pharmaceutical or nutraceutical through manufacture, testing and marketing.

The numerical data obtained from quantitative gene expression and numerical data from calibrated gene expression relative to a baseline profile data set may be stored in databases or digital storage mediums and may retrieved for purposes including managing patient health care or for conducting clinical trials or for characterizing a drug. The data may be transferred in networks via the World Wide Web, email, or internet access site for example or by hard copy so as to be collected and pooled from distant geographic sites (Figure 8).

In a preferred embodiment, a descriptive record is stored in a single or multiple databases where the stored data includes the raw gene expression data (first profile data set) prior to transformation by use of a baseline profile data set, as well as a record of the baseline profile data set used to generate the calibrated profile data set including for example, annotations regarding whether the baseline profile data set is derived from a particular signature panel and any other annotation that facilitates interpretation and use of the data.

Because the data is in a universal format, data handling may readily be done with a computer. The data is organized so as to provide an output optionally corresponding to a graphical representation of a calibrated data set. For example, a distinct sample derived from a subject being at least one of RNA or protein may be denoted as Pᵢ. The first profile data set consists of Mⱼ where Mj is a quantitative measure of a distinct RNA or protein constituent. The record Rᵢ is a ratio of M and P and may be annotated with additional data on the subject relating to for example, age, diet, ethnicity, gender, geographic location, medical disorder, mental disorder, medication, physical activity, body mass and environmental exposure. Moreover, data handling may further include accessing data from a second condition database which may contain additional medical data not presently held with the calibrated profile data sets. In this context, data access may be via a computer network.

The above described data storage on a computer may provide the information in a form that can be accessed by a user. Accordingly, the user may load the information onto a second access site including downloading the information. However, access may be restricted to users having a password or other security device so as to protect the medical records contained within. A feature of this embodiment of the invention is the ability of a user to add new or annotated records to the data set so the records become part of the biological information.

The graphical representation of calibrated profile data sets pertaining to a product such as a drug provides an opportunity for standardizing a product by means of the calibrated profile, more particularly a signature profile. The profile may be used as a feature with which to promote the drug.

The various embodiments of the invention may be also implemented as a computer program product for use with a computer system. The product may include program code for deriving a first profile data set and for producing calibrated profiles. Such implementation may include a series of computer instructions fixed either on a tangible medium, such as a computer readable media (for example, a diskette, CD-ROM, ROM, or fixed disk), or transmittable to a computer system via a modem or other interface device, such as a communications adapter connected to a network over a medium. The medium may be either a tangible medium (for example, optical or analog communications lines) or a medium implemented with wireless techniques (for example, microwave, infrared or other transmission techniques). The series of computer instructions preferably embodies all or part of the functionality previously described herein with respect to the system. Those skilled in the art should appreciate that such computer instructions can be written in a number of programming languages for use with many computer architectures or operating systems. Furthermore, such instructions may be stored in any memory device, such as semiconductor, magnetic, optical or other memory devices, and may be transmitted using any communications technology, such as optical, infrared, microwave, or other transmission technologies. It is expected that such a computer program product may be distributed as a removable medium with accompanying printed or electronic documentation (for example, shrink wrapped software), preloaded with a computer system (for example, on system ROM or fixed disk), or distributed from a server or electronic bulletin board over the network (for example, the Internet or World Wide Web). In addition, a computer system is further provided including derivative modules for deriving a first data set and a calibration profile data set.

### Clinical Trials

The use of calibrated profile data sets for performing clinical trials is illustrated in Figure 10 using the above-described methods and procedures for running a clinical trial or managing patient care. Moreover, standardization between laboratories may be achieved by using a particular indicator cell line such as THP-1 which is stimulated by a known stimulator such as lipopolysaccharide so that resultant profile acts as a measure that the laboratory is performing the protocol correctly.

Examples of how embodiments of the invention may be used for augmenting clinical trials includes providing new methods for patient selection. Clinical trials in which candidate subjects are included or excluded according to a predetermined optimum calibrated profile for a given biological condition can result in more precise monitoring than would be otherwise possible. It can also result in a greater efficiency in clinical trial design because unsuitable patients that have, for example, complicating factors or conditions can be screened out. The calibrated profile data will also enhance the "signal to noise" by removing non-responders from double blind placebo studies. The basic structure of a clinical trial design using gene expression profiling could follow any of several formats. These include testing body fluid from a candidate patient in the trial *ex vivo* against a new therapeutic agent and analyzing the calibrated profiles with respect to an agent-treated and placebo-treated samples using a predetermined panel and evaluating whether the candidate patient would be likely to respond without adverse effects to the composition being tested. In selected indications, profile data obtained from *in vitro* cell cultures or organ cultures may be desired where the cell originates from a target subject or from another subject or from an established cell line, or from a cell samples removed from the target subject where the cell samples may be obtained from any body fluid including a blood, urine, semen, amniotic, or a cerebrospinal fluid sample, or from a scraping from mucosal membranes such as from the buccal cavity, the eye, nose, vagina or by means of a biopsy including epithelial, liver, sternum marrow, testicular, or from tumor tissue removed surgically from a tumor at any location. The above-described sources of samples are applicable to any medical use in which calibrated profile data sets are desired.

*In vitro* dosage and toxicity studies using calibrated profile data sets obtained from indicator cell lines or samples of the patient tested *ex vivo* can provide useful information prior to initiation of the clinical trial and can significantly reduce the cost and time of a clinical trial while increasing the likelihood of identifying the presence of beneficial effect. In particular, the dose can be optimized on an individualized basis to maximize the impact on therapeutic outcome. For example, Figure 12 shows how *ex vivo* blood cells respond to the stimulatory effect of LPS and the subsequent treatment with an anti-inflammatory drug (methotrexate, meclofenamate or methylprednisolone). The data show how the effect of methotrexate and meclofenamate generates similar calibrated profile data sets where the baseline is LPS treated blood. In contrast, the methylprednisolone has a substantially different effect from the other two compounds. A similar type of analysis can be performed with complex mixtures as illustrated in Figure 21 in which the calibrated profiles obtained when Echinacea, Arnica and Siberian Ginseng applied to LPS stimulated blood *ex vivo* are compared. In this example, all three agents appear to act differently from each other with respect to a sample from a single subject. Similar analyses can be used to compare compounds with unknown targets or activities or metabolic patterns to compounds, complex or simple, with known or pre-determined profiles.

The above methods and procedures can be utilized in the design and running of clinical trials or as a supplemental tool. Moreover; the above methods and procedures can be used to monitor the patients' health as well as the patient's responsiveness to an agent before during and after the clinical trial. This includes monitoring whether multiple agents interfere with each other, act synergistically or additively or are toxic or neural with respect to each other. This type of information is very important as individuals take an increasing number of medications.

Similarly, the methods and procedures described above may be used to manage patient care for an individual or a population. Such methods and procedures may also be used to develop a regional or global research network that uses calibrated profile data sets and the resulting databases to conduct research or trials.

Both the calibration profile data sets in graphical form and the associated databases together with information extracted from both are commodities that can be sold together or separately for a variety of purposes. For example, graphic representations of calibration profile data sets can provide a description of a product with respect to its activity that may be used to promote the product. Alternatively, the graphical form of the calibrated profile data sets and access to baseline profile databases provide a means for manufacturers to test discrete batches of product against a gold standard.

The data can be used strategically for design of clinical trials. It can also be useful for physicians practicing at remote sites to offer personalized healthcare to a patient. Accordingly, the physician might set up personalized databases for calibrated profile data sets prior to and after treatment of a particular condition. New data on the subject could be added to the personalized database at each visit to the doctor. The data could be generated at remote sites by the use of kits that permit a physician to obtain a first profile data set on a sample from a patient. For remote users to access the site, it is envisaged that secured access to the global network containing libraries of baseline profile data sets and calibrated profile data sets, classified by particular criteria and representing data from larger populations than a single individual, would be necessary. The access to the global database may be password protected thereby protecting the database from corrupted records and safeguarding personal medical data. The graphical form provided by the calibrated data sets may be used to create catalogs of compounds in a pharmacopiae complete with toxic effects that might arise for particular individuals as well as other types of drug interactions.

Access to the global data base may include the option to load selected data onto a second access site. This process could include downloading the information to whatever site is desired by the user and could include securing hard copies of information. It is desirable to control how and what data is offloaded or dopied to maintain the integrity of the database. It is envisaged that while a global network of clinical data would be an informational resource, it would have utility is conducting research that might include epidemiological studies and studies concerning the mechanism of action of an agent and studies concerning the nature of interpersonal variability as determined by calibrated profile data sets.

### Examples of Medical Uses

(a) Early detection of infectious diseases. Markers or surrogate markers from mice may be obtained for measuring gene expression in humans that indicate early or immediate response to infection, for example, to a virus such as hepatitis virus, or to a bacterium such as *Mycobacterium tuberculosis* (the Gram-positive etiologic agent of tuberculosis) (see Figure 4). Candidate genes are identified and changes in expression of those genes in the presence of a challenge provide a set of markers. The set of markers can combine markers encoded by the genome of the subject and one more distinctive markers encoded by the genome of the infectious agent. For example, changes in expression of an immediate early gene of a virus, e.g. a gene encoding an enzyme of viral replication, and a host gene such as the gene for any or all of IL-2, IL-4 and IL-5, can comprise markers or surrogate markers for a medical condition capable of detecting that condition prior to the onset of medical symptoms. This method affords earlier detection of an infection than is possible using current diagnostic techniques.
(b) Toxicity profiles and mechanistic profiles obtained from an *in vitro* assay and *in vivo* assays. Toxicity and mechanistic information arising from the administration of a compound to a population of cells can be monitored using calibrated profile data sets. The following is an example of an experimental protocol for obtaining this information. Firstly, an experimental groups is established: (1) control cells maintained without therapeutic agent and without stimulus; (2) cells treated with therapeutic agent but without stimulus; (3) cells without therapeutic agent but with stimulus, (4) sample with therapeutic agent and with stimulus. The population of cells can be selected from primary cell cultures prepared in culture plates using methods well established in the art; or mature differentiated cell preparation from whole blood or isolated monocytes from the target organism, which in this example is mouse.
   The cells are stimulated so as to present a targeted physiological condition by pretreatment with LPS purified from a Gram-negative bacterium (a variety of LPS preparations from pathogenic bacteria, for example, from *Salmonella typhimurium* and from *Escherichia coli* O1157:H7, are available from Sigma, St. Louis, MO). The therapeutic agent administered to the cell samples in this example is an inhibitor of an enzyme known to be key in disease etiology, namely an inhibitor of a protease or a nucleic acid polymerase. Following treatment by addition of the therapeutic agent and further incubation for four to six hours, samples of the cells are harvested and analyzed for gene expression. Nucleic acid, specifically RNA, can be prepared from the sample by methods known to one or ordinary skill in the art (see, for example, the Lyse-N-Go^{™} reagent, Pierce Chem. Co., Rockford, IL). Samples are analyzed by QPCR according to a quantitative replicative procedure, (quantitative polymerase chain reaction procedure (QPCR)) (see, for example, Gibson,U. 1996 Genome Res.6:995-1001, and references cited therein). Total RNA was assessed using universal primers. Toxicity of the agent for cells can be measured in untreated cells by vital stain uptake, rate of DNA synthesis (autoradiography of labeled nucleic compared to cells stained), stain by DNA-specific eyes (Hoechst), etc. Mechanistic profiles can be determined by analysis of the identifies of *de novo* up- or down-regulated genes. Further, in the presence of a therapeutic agent, some genes are not expressed, indicating potential efficacy of the therapeutic agent in suppressing the effects of stimulation by the LPS. For example, in Figure 21, levels of ICE that are somewhat stimulated in the presence of LPS +Echinacea are substantially depressed by LPS + Arnica relative to LPS stimulated cells absent agent. Levels of HSP 70 which are depressed in the presence of LPS+Echinacea are substantially stimulated in the presence of LPS +Arnica, and LPS + Siberian Ginseng relative to LPS stimulated cells absent the addition of an agent. Levels of IL-12p40 which are slightly increased in the presence of LPS + Echinacea are substantially depressed in the presence of LPS + Arnica and LPS +Siberian Ginseng relative to LPS stimulation. In contrast to the above using nutraceuticals, Figure 16 shows a much enhanced reduction of gene expression in whole blood for IL-1a, Il-1b, Il-7, Il-10, IL-IL-15, IFN-g, TGF-b, TNF-b cox-2, and ICAM in the presence of prednisolone +LPS when compared to arnica +LPS or nothing +LPS.
(c) Quantitation of gene expression in a blood cell to predict toxicity in another tissue or organ.
   Leukocytes can be obtained from a blood sample of a subject, for the purpose of assessing the appearance of a pathological condition in another organ, for example, the liver. A profile data set is obtained of genes expressed in the leukocytes, for example, genes encoding a set of lymphokines and cytokines. The data set is compared to that of the database, to examine correlations for example to other subjects, and to the subject prior to administration of a therapeutic agent.
   By this method, a correlation can be drawn between, for example, administration of acetaminophen (Tylenol) and sensitivity to this therapeutic agent and manifested by liver damage. An early prediction of therapeutic agent sensitivity, detected prior to the onset of actual damage to the liver, can be clinically available so that the subject receives no further administration of acetaminophen. The success of the database is the ability to detect a correlation or correlations prior to the onset of traditional medical assessments, such as increase in bilirubin level or other indication of liver pathology.
(d) Calibrated profiles from blood cells for prognosis of severity and prediction of adverse reactions in treatment of an autoimmune disease.
   The probability and timing of onset of symptoms of an autoimmune disease, for example, rheumatoid arthritis, may be monitored by appearance of expression of markers or surrogate markers as determined by the methods of gene expression profiling of markers or surrogate markers and comparison to a profile database as described above. Thus an indication of imminent onset can be obtained, and advance management by utilization of preventive measures to forestall onset, can be taken. Further, the user can choose a set of potential therapeutic agents, and assess for a given agent, the probability that a subject will present an adverse reaction if given a full course of treatment, prior to that full course. For example, using embodiments of the invention, a single dose of the agent methotrexate can be administered to a subject having arthritis and in need of a therapeutic agent. If the gene expression profile data set of the subject in response to a single dose of methotrexate correlates with data sets from subjects having adverse reactions to this agent, then administration of a full course of methotrexate is counterindicated. Conversely, if the gene expression profile data set correlates with those of subjects who have responded positively to administration of a course of methotrexate treatment, then this therapeutic agent can be administered to the subject with much lower probability of adverse reaction.

### Discussion of Figures

Figures 1-4 illustrate some of the applications of calibrated profile data sets. In Figure 1, three possible scenarios are provided. Firstly, a candidate therapeutic agent may be tested to determine its molecular pharmacology and toxicology profiles. The test might include obtaining calibrated profile data sets for a series of panels selected on the basis of what activity is predicted for the drug. The population of cells exposed to the agent may be the result of *in vivo* administration as depicted by the mouse or direct exposure *in vitro* where the cells may be an indicator cell line or an *ex vivo* sample from the subject. The result of the screen is the identification of more effective drug candidates for testing in human subjects.

The second scenario in Figure 1 is the use of calibrated profile data sets to identify a suitable clinical population for screening a potential therapeutic agent. Both demonstration of lack of toxicity and demonstration of clinical efficacy require certain assumptions about the clinical population. The calibrated profile data sets provides a means for establishing those assumptions with respect to the biological condition of the individuals selected for the clinical trials.

The third scenario in Figure 1 is the opportunity to practice individualized medicine which may include creating an archive of calibrate profile data sets on the individual in a state of health such that changes can be identified using signature panels so as to permit prognosis or diagnosis of a particular condition. Moreover, stored information about the patient in the form of calibrated profile data sets permits selecting one of a group of possible therapeutic agents most like to be effective for the patient, optimizing dosage of drug and detecting adverse effects that might arise through drug interactions before symptoms arise. The result of the use of calibrated profile data sets is to provide more efficient and cost effective health care management.

The novel approach described above for evaluating a biological condition of a subject may be applied to an *ex vivo* or *in vitro* assay for measuring the effect of an agent on a biological condition as illustrated in Figures 2-4. A sample from the patient may be measured directly *ex vivo* or tested *ex vivo* against an agent to predict an effect in the patient. This provides a quick and effective way to determine which drug chosen from within a single class of drugs that all may be used to treat a particular condition, may be most effective for a given subject. Alternatively, an agent may be tested on an indicator cell line that can provide a quantitative measure of therapeutic performance in a class of individuals.

Figure 2 illustrates how calibrated profile data sets may assist in screening a library of candidate compounds to discover candidate drugs. Starting with, for example, 500 candidate drugs, these can be tested in indicator cells or *ex vivo* body fluid or tissues against signature panels for *in vitro* toxicology or metabolic indicators. The figure illustrates the large number of compounds that entered in late stages in the development process only to ultimately be rejected due to adverse biological interactions. It is expected that early adoption of the use of calibrated profile data sets will more readily identify likely successful candidates and thereby reduce the expense and untoward effects of animal and human experimentation for compounds that could have been predicted to fail.

Figure 3 describes multiple screens in which a compound might be administered to an experimental animal such as a mouse or to an indicator cell line. The *in vivo* or *ex vivo* or indicator cell sample might further be treated with a stimulus. The result of both the compound and the stimulus could then be detected using to signature profiles for toxicity or for mechanism to compare the effect of no drug +/- stimulus or +/-drug and no stimulus. Both *in vitro* (left panel) and *in vivo* (right panel) studies can be used to evaluate the effect of a compound (drug, nutraceutical, environmental stimuli, etc.). The right hand panel also illustrates the specific embodiment of an "*in vitro* clinical trial", that is, treatment of cells obtained from a subject and treated with a compound (with or without a stimulus) *in vitro* (or *ex vivo*) in order to predict the outcome of similar treatment of the subject *in vivo* (see Fig. 15 for a specific example). The output from both panels is described as toxicity and mechanistic profiles. Either experimental course may be used to both evaluate potential toxicity, e.g., using the toxicity, or liver metabolism panels, and to determine or confirm likely mechanism of action by a critical selection of a gene panel(s) that illustrates and differentiates molecular mechanisms of action (see Figure 12 for a specific example).

Figure 4 illustrates a bioassay in which cells are removed from the subject and tested *ex vivo* with the addition of a compound and also a challenge or stimulus. The *ex vivo* effect of stimulus and then drug on whole blood taken from a human subject is shown in Figure 12 in which the stimulus is lipopolysaccharide (an inflammatory agent) while the drug is any of methotrexate, meclofenamate or methylprednisolone using a signature panel for inflammation. Methylprednisolone, a drug commonly used in the treatment of acute exacerbations of COPD as well as in the chronic management of this disease, is considered to be a potent by non-specific anti-inflammatory agent. However, as demonstrated in Figure 22, its effects on gene expression are dependent on the stimulus. While there are general qualitative similarities between the effects on gene expression across these three stimuli, there are both quantitative and qualitative differences that may be important in understanding when glucocorticoid intervention is warranted.

According to embodiments of the invention, an indicator cell population is used to measure quantitative gene expression the effect of an agent or a biological sample may influence the choice of which indicator cell line will be most informative. For example, a cloned cell line such as THP-1 or a primary cell population (peripheral mononuclear cells) may provide information that is comparable to that obtained from a body sample directly (see Figure 15). The normal state of gene expression may range from zero or few transcripts to 10⁵ or more transcripts.

Similarly, an agent may be evaluated for its effect on any population of cells, either *in vivo, ex vivo* or *in vitro,* by administering the agent and then determining a calibrate profile data set for those cells under the selected conditions. Examples of this approach are provided in Figures 10-16 and 18. Figure 18 further provides calibrated profile data sets for different concentrations of a single agent showing that the transcription of selected constituents vary with dose and therefore the anticipated effectiveness with respect to the biological condition.

The above description of determining a biological condition is exemplified as follows. The action of a pharmaceutical or nutraceutical is measured with respect to its anti-inflammatory properties. The measurement of the effect may be established using a panel of constituent gene loci, for example, an inflammation panel, including, Interleukin 1 alpha (IL-1α) or Tumor Necrosis Factor alpha (TNF-α). The anti-inflammatory effect may first be established by treating indicator cells or sample cells *ex vivo* with a known inflammation inducers (for example, lipopolysaccharide or other mitogens) followed by treatment with the experimental agent or condition expected to suppress or reduce the expression from the appropriate gene loci. According the baseline profile data set is the delta change in gene expression for a particular panel of constituents. The addition of a potential anti-inflammatory agent results in a second delta change that is superimposed on a first delta change. This is illustrated for example in Figure 12. Methylprednisolone has a substantial down regulation effect on IL-2 in blood cells stimulated *ex vivo* with LPS where the baseline data set is LPS stimulated cells. In this case there is a negative delta. In contrast, IL-2 appears to be upregulated in whole blood not previously exposed to LPS, where the baseline data set is unstimulated cells. (Figure 16b) This is consistent with the observation that methylprednisolone stimulated IL-2 production.

The determination of the biological condition of a subject may include measuring and storing additional data about the subject. For example, if the subject is a human or mammalian patient, additional clinical indicators may be determined from blood chemistry, urinalysis, X-ray, other chemical assays and physical or sociological findings.

Figure 7 illustrates how the accumulation of calibrated profile data sets can improve the predictive power of the database and thereby increase its value in generating information about a biological condition or agent. The figure indicates the use of the database in terms of its predictive power to, for example, predict the course of a therapeutic intervention, follow the course of an individual subject compared to a population, prediction of a likely mechanism of metabolism or molecular mechanism of action or a comprehensive database that allows comparison of a single profile to a collection of signature, calibrated precision profiles.

Preferred embodiments of how the database may be used is provided in Figure 8. Figure 8 illustrates display of a data profile set from the source database. Entries for input include a name, an Experimental Type, and whether the entry is a New Reference; Cell/Tissue/Species and whether these are new; Therapeutic agent (compound), Dose, and additional parameters and whether the therapeutic agent is new. Observations are recorded according to the identity of a Gene (New Gene) and a Protein (New Protein). The Stimulus or other Treatment, if any, and the Dose are entered. Gene (and/or Protein) Expression, Expression Value, Expression Units if appropriate and Expression Time are shown. The figure specifically illustrates the range of applicable fields of investigation from complex natural products to clinical trails in humans, linkage to traditional forms of measurement and evaluation such as literature citations, clinical indicators and traditional pharmacokinetic measurements. Expert analysis of the precision profile data contained in the database may then be used to guide product development and marketing, or used to improve the clinical decision making concerning the health of a single individual or population of individuals.

It is anticipated that one form of record might provide information about a subject or agent with respect to identity, medical history including traditional pharmaceutical/medical data, clinical indications as determined from literature data, reference to additional types of analysis in the database, etc.

Figure 9 shows an embodiment of the present invention in which profile data is evaluated using data from a database that is remotely accessed over a network. The figure illustrates that data are expected to be derived at one or more locations, compared using a central database and information obtained used to affect, for example, the course of treatment of an individual or population. The two-way nature of 1109 illustrates the iterative process whereby the database affects the course of treatment or development, and outcome or response to such intervention again becomes part of the database. In a first location, as in Figure 5, from a tissue sample procured in box 1101, there are derived multiple RNA species pursuant to box 1102, and then in box 1103, profile data are quantified to produce a profile data set that is pertinent to the tissue sample obtained in box 1101. In order to evaluate the profile data set, in box 1104 information is retrieved from database 1108, which is located in a second location. In fact the database may be in communication with a large number of locations, each of which is generating profile data that must be evaluated. The retrieval of information from the database is accomplished over a network 1109, which may include the Internet, in a manner known in the art. Once information has been obtained from the database 1108, the information is used in evaluating the quantified profile data in box 1105, with the result in box 1106 that the medical condition of the subject may be assessed. The database 1108 is in box 1107 updated over the network 1109 to reflect the profile data that have been quantified in box 1103. In this manner the database 1108 may be updated to reflect the profile data obtained over all locations, and each location has the benefit of the data obtained from all of the locations.

### EXAMPLES

### Example 1. (a) Use of whole blood for ex vivo assessment of a biological condition affected by an agent.

Human blood is obtained by venipuncture and prepared for assay by aliquoting samples for baseline, no stimulus, and stimulus with sufficient volume for at least three time points. Typical stimuli include lipopolysaccharide (LPS), phytohemagglutinin (PHA) and heat-killed staphylococci (HKS) or carrageean and may be used individually (typically) or in combination. The aliquots of heparinized, whole blood are mixed without stimulus and held at *37*°C in an atmosphere of 5% CO2 for 30 minutes. Stimulus is added at varying concentrations, mixed and held loosely capped at 37°C for 30 min. Additional test compounds may be added at this point and held for varying times depending on the expected pharmacokinetics of the test compound. At defined times, cells are collected by centrifugation, the plasma removed and RNA extracted by various standard means.

### (b) Preparation of RNA for Measuring Gene Expression.

Nucleic acids, RNA and or DNA are purified from cells, tissues or fluids of the test population or indicator cell lines. RNA is preferentially obtained from the nucleic acid mix using a variety of standard procedures (or RNA Isolation Strategies, pp.55-104, in RNA Methodologies, A laboratory Guide for Isolation and Characterization, 2nd edition,1998,Robert E. Farrell, Jr., Ed., Academic Press); in the present use using a filter-based RNA isolation system from Ambion (RNAqueous^{™}, Phenol-free Total RNA Isolation Kit, Catalog #1912, version 9908; Austin, Texas). Specific RNAs are amplified using message specific primers or random primers. The specific primers are synthesized from data obtained from public databases (e.g., Unigene, National Center for Biotechnology Information, National Library of Medicine, Bethesda, MD), including information from genomic and cDNA libraries obtained from humans and other animals. Primers are chosen to preferentially amplify from specific RNAs obtained from the test or indicator samples, see, for example, RT PCR, Chapter 15 in RNA Methodologies, A Laboratory Guide for Isolation and Characterization, 2nd edition, 1998,Robert E. Farrell, Jr., Ed., Academic Press; or Chapter 22 pp.143-151, RNA Isolation and Characterization Protocols, Methods in Molecular Biology, Volume 86,1998, R Rapley and D. L. Manning Eds., Human Press, or 14 in Statistical refinement of primer design parameters, Chapter 5, pp.55-72, PCR Applications: Protocols for Functional Genomics, M.A.Innis, D.H. Gelfand and J.J. Sninsky, Eds., 1999, Academic Press). Amplifications are carried out in either isothermic conditions or using a thermal cycler (for example, a ABI 9600 or 9700 or 7700 obtained from PE Biosystems, Foster City, CA; see Nucleic Acid Detection Methods, pp.1-24, in Molecular Methods for Virus Detection, D.L.Wiedbrauk and D.H., Farkas, Eds.,1995, Academic Press). Amplified nucleic acids are detected using fluorescent-tagged detection primers (see, for example, Taqman^{™} PCR Reagent Kit, Protocol, part number 402823 revision A, 1996, PE Applied Biosystems, Foster City CA.) that are identified and synthesized from publicly known databases as described for the amplification primers. In the present case, amplified DNA is detected and quantified using the ABI Prism 7700 Sequence Detection System obtained from PE Biosystems (Foster City, CA). Amounts of specific RNAs contained in the test sample or obtained from the indicator cell lines can be related to the relative quantity of fluorescence observed (see for example, Advances in Quantitative PCR Technology: 5' Nuclease Assays, Y.S. Lie and C.J. Petropolus, Current Opinion in Biotechnology,1998, 9:43-48, or Rapid Thermal Cycling and PCR Kinetics, pp. 211-229, chapter 14 in PCR Applications: Protocols for Functional Genomics, M.A.Innis, D.H. Gelfand and J.J. Sninsky, Eds., 1999, Academic Press.

### Example 2. Different inflammatory stimuli give rise to different, baseline profile data sets so that the calibrated precision profiles for different agents in the same class of anti-inflammatory result in different signature profiles.

Figure 11 documents the usefulness of different inflammatory stimuli to give rise to different, baseline profile data sets so that the calibrated precision profile data sets for the three anti-inflammatory agents tested result in different signature profiles. The different profiles reflect the difference in the molecular targets and mechanisms of action of the three agents derived from a single class of therapeutics, anti-inflammatory agents. The figure also illustrates the extraordinary range of detection (y-axis) from less than 10 fold difference from the calibrated profile to plus or minus 10E13 increase or decrease in gene expression when compared to the calibrator. Comparison to the calibrator results in gene expression profiles that are increased, decreased, or without change from the calibrated set.

Figure 11(a) shows relative gene expression (mRNA synthesis) in heat-killed staphylococci (HKS)-stimulated cells, and the effect of three different compounds (TPCK, UT-77, and "Dex", or dexamethasone). Compound TPCK caused a 10-fold decrease in relative IFN-γ expression, and 100,000-fold decreases in IL-4 and IL-5 expression. Further, compound UT-77 caused even greater magnitude of increases in relative expression of the gene encoding IL-5, and more modest increases in IL-1 expression (more than 10-fold) and IFN-γ. Such effects can be highly significant in disease etiologies and outcomes, and have predictive value concerning the usefulness as therapeutic agents of these compounds or similar chemical entities or chemicals that act similarly. HKS cells are an *in vitro* model of Gram-positive bacterial infection.

Fig. 11(b) displays analyses of expression of the 12 genes in lipopolysaccharide-(LPS)-treated cells, an *in vitro* model of Gram-negative bacterial infection. These data include several striking contrasts to the data in Fig. 11(a). Thus treatment with the therapeutic agent Dex caused a striking decrease in expression of the IL-2 gene in LPS-treated cells, and a striking increase in IL-2 expression in HICS-treated cells. Strikingly large differences in gene expression in the differently stimulated cells can be seen for the IL-4 and the IL-5 genes. Expression of the gene for IFN, in contrast, responded similarly in cells treated by either of the stimuli and any of the therapeutic agents.

By these criteria, expression of the genes for IL-2, IL-4 and IL-5 were observed to be candidate markers or surrogate markers in cell model systems to distinguish responses of the cells to Gram-positive and Gram-negative bacterial infection.

### Example 3. A single therapeutic agent for treating a particular condition can be differentiated from a second therapeutic agent that also treats the particular condition by a signature profile for a given panel of gene loci.

Figure 12 shows a calibrated profile data set for a panel having 8 constituents that are indicative of a biological condition that includes inflammation. The profiles are shown for three different anti-inflammatory agents-methotrexate, meclofenamate and methylprednisolone. The calibrated profile data sets for each agent as shown represents a signature profile for that agent. This signature profile may serve as a device for establishing quality control for a batch of the agent. Indeed, it is envisaged that compounds or classes of compounds on the market or in development may be characterized by a signature profile. The signature profile may be represented in a graphical format, more particularly as a bar graph as provided in Figure 12. For Figure 12, an *ex vivo* sample was tested. A sample of blood was taken from the subject. Aliquots of the sample were subjected to lipopolysaccharide (LPS) *ex vivo.* After 30 minutes, the anti-inflammatory agent as indicated was added to an aliquot of the sample of blood and after about another 4 hours, the expression of the panel of genes (Il-1a, Il-2, Il-8, Il-10, Il-12p35, Il-12p40, IL-15, IFN-Gamma and TNF-a) was determined. Although the calibrated profile of methotrexate and meclofenamate were similar, the calibrated profile of methylprednisolone was substantially different. Differences may be reflective of the differences of the mechanisms or target(s) of action of this agent within the general class of anti-inflammatory compounds. The baseline is the profile data set for lipopolysaccharide absent any additional agents.

### Example 4. There is relatively low variability with respect to the profile within a single individual over time when the calibrated precision profile is determined from the measurement of gene expression across many gene loci that have been appropriately induced.

Figure 13(a)(b) and (c) show a graphical representation of calibrated precision profile data sets for two different samples of whole blood. Heparinized whole blood from a single normal healthy volunteer was collected on two separate occasions of more than 2 weeks apart. Figure 13a for sample 991116 and Figure 13b,for sample 991028 reflect the biological condition of the tested cells from the single donor using a panel (i.e., the inflammation panel) of 24 members, in response to stimulation with one of three different agents. The baseline in this example is derived from untreated cells obtained from the same individual. The calibrated profiles are shown for cells exposed for 4 to 6 hours to lipopolysaccharide (LPS), heat-killed Stapylococci (HKS), and phytohemagglutinin (PHA). Figure 13c shows a direct comparison of LPS-stimulated blood sample 99116 with respect to blood sample 991028, i.e., 991028 is used as the calibrator or baseline profile data set. The messenger RNA levels measured on 10/28/99 were used to compare the levels of messenger RNA measured on 11/16/99. A perfect identity of RNA levels would be represented by a flat line at unity. These data clearly show that for baseline gene expression, there can be as much as an 8 fold difference (c-jun) in messenger RNA levels. However, for most of the genes measured, the levels of messenger RNA measured on one day are within 2-3 fold of those measured on a different day. 13(d) is similar to 13(c) except that the cells were not stimulated with LPS.

The figure documents the relatively low variability with respect to the profile within a single individual over time when the calibrated precision profile is determined from the measurement of gene expression across many gene loci that have been appropriately induced. The figure illustrates (1) the class-specific effects (generally inflammatory as determined by the effect on pro-inflammatory gene loci, e.g., TNF-alpha, IL-1 alpha and IL-1 beta), (2) the agent-specific effects quantitative differences between each of the agents at the same gene loci (e.g., IL-2) and (3) reproducible and therefore predictable effects on the subject population, TK (Figure 13c)

### Example 5. Similarities and differences in the effect of a single agent on cell populations differing in their biological condition.

*Ex-vivo* gene expression analysis can be performed by obtaining the blood of a subject for example by drawing the blood into a vacutainer tube with sodium heparin as an anticoagulant. An anti-inflammatory such as 3-methylprednisolone at a final concentration of 10 micromolar was added to blood in a polypropylene tube, incubated for 30 minutes at 37C. in 5% CO₂. After 30 minutes a stimuli such as LPS at 10 ng/mL or heat killed staphlococcus (HKS) at 1:100 dilution was added to the drug treated whole blood. Incubation continued at 37C. in 5% CO₂ for 6 hours unless otherwise indicated. Erythrocytes were lysed in RBC lysis solution (Ambion) and remaining cells were lysed according to the Ambion RNAqueous-Blood module (catalog # 1913). RNA was eluted in Ambion elution solution. RNA was DNAsed treated with 1 unit of DNAse I (Ambion #2222) in 1X DNAse buffer at 37C. for 30 minutes. First strand synthesis was performed using the Perkin-Elmer TaqMan Reverse Transcriptase kit with MultiScribe reverse transcriptase (catalog # N808-0234). Quality check of RT reactions were performed with Taqman PCR chemistry using the 18S rRNA pre-developed assay reagents (PDAR) from PE Biosystems (part #4310893E). PCR assay was performed on 6 to 24 genes in four replicates on the PE Biosystems 7700. PCR assays were performed according to specifications outlined with the PDAR product. Relative quantitation of the gene of interest was calibrated against 18S rRNA expression as described in PE product User Bulletin 2 (1997) and elaborated in Hirayama, et al (Blood 92,1998:46-52) using 18S instead of GAPDH.

Relative quantitation of the mRNA was measured by the difference in threshold cycles between 18S and the gene of interest. This delta C_{T} was then compared to the normalizing condition, either subject before treatment, or stimuli without drug in an *ex-vivo* assay to measure "fold induction" represented in the bar graphs. (Figure 14) For example in the above graph, IFN-levels are 1 /50 less on day 3 than before treatment.

### Example 6. In vivo and Ex vivo samples provide comparable signature profiles.

Figure 15 shows the calibrated profile data set for two subjects (Subject 1 and Subject 2) who have been treated over a three day period with a standard dose of the corticosteroids, dexamethasone. Blood from each subjects was obtained 72 hours later and a quantitative measure of the amount of RNA corresponding to the panel constituents was determined. Although, the calibrated profile data set for each subject was similar for most gene loci, some notable differences were also detected, for example for Il-2, Il-10, Il-6 and GM-CSF. A calibrated profile data set is also shown for comparison for an *ex vivo* sample of blood from sample 1 prior to treatment with corticosteroid where the *ex vivo* sample is subjected to an equivalent amount of corticosteroid *in vitro* as calculated to be the plasma level in the subject. The similarity in the calibrated profile data set for *ex vivo* samples when compared to *in vivo* samples provides support for an *in vitro* assay that will predict the *in vivo* action of the compound. We have observed a similar comparable effect between *in vivo* and *ex vivo* samples infected with an infectious agent, more particularly bacterial or viral agents. We have concluded therefore that the *ex vivo* samples provide an effective method of determining the effect of a single compound or multiple compounds on a patient, where the multiple compounds may be either used in combination, in parallel or sequentially to optimize the selection of an agent for a biological condition for the subject.

### Example 7. Demonstration of reproducibility of an in vitro response with an approved anti-inflammatory on 5 different donor subjects.

Comparison and analysis of the Figures 18a through 18e demonstrates the consistency of effect of the stimulus and *in vitro* treatment with an approved anti-inflammatory on 5 different donors (each figure representing a unique donor). The use of a known and tested stimulus results in a highly reproducible gene response *in vitro* that may be correlated with a predictable *in vivo* response. Figures 18a-18e provide the results of analysis of 5 donors from which a blood sample has been taken. The blood samples were exposed to a therapeutic agent at various concentrations ranging from 0.1µM to 5µM, more particularly 0.1µM, 0.3µM, 1µM, 3µM and 5µM, for a 4 hour period. Different concentrations of the drug resulted in a calibrated profile data set for an inflammation panel at each concentration that was qualitatively different from the next Figure 18a corresponds to donor 1, Figure 18b corresponds to donor 2, Figure 18c corresponds to donor 3, Figure 18d corresponds to donor 4 and Figure 18e corresponds to donor 5. Each individual varied from the other and also provided a variable profile for a different concentration. This set of figures illustrates the high level of information obtainable by calibrated profile data sets.

### Example 8. A calibrated profile data set may provide a signature profile for a complex mixture of compounds.

Figure 21 illustrates the effect of three different anti-inflammatory herbs on a panel of constituents including constituents of an inflammatory panel (TNF-a, Il-1b, ICAM, Il-8, Il-10, Il-12p40, ICE, cox-2, cox-1 and mmp-3) a cell growth and differentiation panel (c-fos, c-jun and STAT3) , a toxicity panel (SOD-1, TACE, GR, HSP70, GST, c-fos, c-jun, INOS) and a liver metabolism panel (INOS, cyp-a and u-pa). The cells assayed in Figure 21 are aliquots of blood from a subject that are exposed *ex vivo* to lipopolysaccharide and to Echinacea (SPM9910214) Arnica (SPM9910076) and Siberian Ginseng (SPM9910074), each of the nutraceuticals being applied to the blood sample at the same concentration of 200ug/ml. The baseline is cell sample with lipopolysaccharide in the absence of a nutraceutical. Each nutraceutical (formed from a complex mixture) has a characteristic signature profile just as did the single compound pharmaceutical anti-inflammatory agents. The signature profile may be provided in a graphic form that can be use to identify a herbal while providing information concerning its properties and its efficacy for a single subject or for an average population of subjects.

### Example 9. A quality control assay for Echinacea brands using calibrated profile data sets.

Figure 24 shows a graphic representation of the calibrated profile data sets for four different commercial brands of Echinacea Brands using an Inflammation Panel. As expected, SPM007 and SPM003 gave the signature, calibrated profiles similar to authentic Echinacea samples SPM010 and SPM 016, although labeled and sold as Echinacea when tested using the system described in Figure 14, resulted in signature calibrated profiles that were substantially similar to the profile obtained with lipopolysaccharide alone. Echinacea samples SPM010 and SPM016 were found to have elevated, highly biologically active levels of endotoxin while the LPS levels in SP007 and SP003 were undetectable. A stored signature profile for active echinacea obtained from a panel designed to test efficacy and mode of action, e.g., the inflammation panel, permits evaluation of new batches of Echinacea, differentiation of existing or new brands of Echinacea, guide the isolation and development of new compounds with different or similar activities from a complex compound like Echinacea or may be used in the development of quality assurance in the production, analysis and sale of new or previously marketed compounds. In the example cited, two of the brands of Echinacea SP010 and SP016.result in calibrated profiles that are characteristic of authentic Echinacea.

### Example 10. Comparison of three herbal preparations using an indicator cell line.

Figures 25 (a)-(c) provide calibrated profile data sets for three herbal preparations with respect to an indicator cell line (THP-1) rather than a blood sample from a subject. In Figure 25(a), the baseline is the profile data set for THP-1 cells absent the herbal while the histograms represent the calibrated profile data sets for the same herbal from three different manufacturing sources of the same herb at 250ug/ml. Gene expression results are shown on a log scale. Similar to the observation in Figure 14, these demonstrate that similarly labeled compounds obtained from different sources have demonstrable and quantifiable differences in calibrated profiles using a specific panel, eg. the inflammation panel designed to obtain information about the expression of gene products related to inflammation and infection. This suggests that the compounds likely have different efficacies when used for specific purposes.

Figure 25(b) provides a comparison of the calibrated profile of a single herb at three concentrations using the indicator cell line of THP-1. The baseline profile data set is untreated THP-1 cells. Analysis of the data suggests a concentration-dependent response in the indicator cell lines which, although demonstrated here, may be indicative of a similar response in subjects.

Figure 25(c) provides a comparison of four commercial Echinacea brands used at the same concentration and tested against a panel of constituents using a THP-1 cell line as an indicator cell population. Differential expression, as revealed by differences in the calibrated profiles, allows direct comparisons of complex compounds to be made. For example, analysis of the differences in the calibrated profiles could be used to guide compound isolation and development, product differentiation in the marketplace, or used by the consumer or health professional to guide the individualized choice of a single compound from a class of similar compounds that may be suited for a particular biological condition.

## Claims

1. A method for evaluating a biological condition of a subject, based on a first sample obtained from the subject, the sample providing a source of RNAs; **characterised in that** the method comprises:
(a) deriving from the first sample a first profile data set, which first profile data set comprises 3 or more members, each member being a relative quantitative measure of the amount in said first sample of a distinct transcribed RNA constituent in a panel of constituents selected such that measurement of said constituents enables evaluation of said biological condition, the relative quantitation of mRNA in said sample being determined by performing quantitative PCR on RNA extracted from the sample with a defined efficiency of amplification for the target transcripts and determining the difference in threshold cycles (delta C_{T}) between a size marker and the target transcripts of interest; and
(b) producing a calibrated profile data set for the panel, each member of the calibrated profile data set being a function of a corresponding member of the first profile data set and a corresponding member of a baseline profile data set for the panel; wherein the calibrated profile data set represents a set of values in which a pattern of variation correlates in a reproducible manner with a particular condition.

2. A method according to claim 1, wherein it further comprises distinguishing between two biological conditions by performing (a) and (b) for each of the two biological conditions

3. A method according to claim 1, wherein the function includes a ratio of such members.

4. A method according to claim 3, wherein the function includes a logarithm of the ratio.

5. A method according to any of claims 1 to 4, wherein the biological condition is a complex disease process involving multiple genes, the disease being of a type involving at least one of inflammation, auto-immune disease, degenerative disease, allergy, vascular disease, ischemia, cancer, developmental disease, hormonal condition, aging and infectious disease.

6. A method according to claim 5, wherein the condition is **characterised by** inflammation and the panel includes at least one of Il-1 alpha, Il-1 beta, Il-2, Il-4, Il-5, Il-6, Il-8, Il-10, Il2p40, Il-18, GMCSF, IFN-gamma, TGF-b, cox-1, cox-2, ICE, mmp-3, mmp-9, ICAM, TNF-a and TNF-b.

7. A method according to claim 1, wherein the sample is a blood sample.

8. A method according to any of claims 1 to 4, wherein the condition is **characterised by** liver metabolism and the panel of constituents is a liver metabolism panel.

9. A method according to any of claim 1 to 4, wherein the biological condition is a toxicity and the panel of constituents is a toxicity panel.

10. A method according to claim 9, wherein the toxicity is an adverse reaction to a drug.

11. A method as claimed in claim 1, wherein said first profile data set comprises at least 5 members.

## Patentansprüche

1. Ein Verfahren zur Beurteilung eines biologischen Zustands eines Testobjekts auf der Basis einer ersten Probe, die von dem Testobjekt erhalten wird, wobei die Probe eine Quelle von RNAs bereit stellt; **dadurch gekennzeichnet, dass** das Verfahren umfasst:
(a) Ableiten aus der ersten Probe eines ersten Profildatensatzes, wobei der erste Profildatensatz 3 oder mehr Elemente umfasst, wobei jedes Element ein relatives quantitatives Maß für die Menge in der ersten Probe eines gesonderten transkribierten RNA-Bestandteils in einer Gruppe von Bestandteilen ist, die derart ausgewählt werden, dass die Messung der Bestandteile die Beurteilung des biologischen Zustands ermöglicht, wobei die relative Quantifizierung der mRNA in der Probe bestimmt wird, indem eine quantitative PCR mit RNA, welche aus der Probe extrahiert wird, mit einer definierten Amplifikationseffizienz für die Targettranskripte durchgeführt wird und der Unterschied bei Grenzwertzyklen (delta C_{T}) zwischen einem Größenmarker und den Targettranskripten von Interesse bestimmt wird; und
(b) Erzeugen eines kalibrierten Profildatensatzes für die Gruppe, wobei jedes Element des kalibrierten Profildatensatzes eine Funktion eines entsprechenden Elements des ersten Profildatensatzes und eines entsprechenden Elements eines Grundlininenprofildatensatzes für die Gruppe ist; wobei der kalibrierte Profildatensatz einen Satz von Werten darstellt, bei welchem ein Variationsmuster in einer reproduzierbaren Weise mit einem bestimmten Zustand korreliert.

2. Ein Verfahren gemäß Anspruch 1, wobei dieses weiterhin das Unterscheiden zwischen zwei biologischen Zuständen durch ein Ausführen von (a) und (b) für jeden der zwei biologischen Zustände umfasst.

3. Ein Verfahren gemäß Anspruch 1, wobei die Funktion ein Verhältnis solcher Elemente einschließt.

4. Ein Verfahren gemäß Anspruch 3, wobei die Funktion einen Logarithmus des Verhältnisses einschließt.

5. Ein Verfahren gemäß irgendeinem der Ansprüche 1 bis 4, wobei der biologische Zustand ein komplexer Krankheitsprozess ist, an dem mehrere Gene beteiligt sind, wobei die Krankheit zu einem Typ gehört, an dem wenigstens ein Element von Entzündung, Autoimmunkrankheit, degenerativer Krankheit, Allergie, Gefäßkrankheit, Ischämie, Krebs, Entwicklungskrankheit, Hormonstörung, Alterung und Infektionskrankheit beteiligt ist.

6. Ein Verfahren gemäß Anspruch 5, wobei der Zustand durch eine Entzündung **gekennzeichnet** ist und die Gruppe wenigstens einen von Il-1 alpha, Il-1 beta, Il-2, Il-4, Il-5, Il-6, Il-8, Il-10, Il2p40. Il-18, GMCSF. IFN-gamma, TGF-b, cox-1, cox-2, ICE, mmp-3, mmp-9, ICAM, TNF-a und TNF-b beinhaltet.

7. Ein Verfahren gemäß Anspruch 1, wobei die Probe eine Blutprobe ist.

8. Ein Verfahren gemäß irgendeinem der Ansprüche 1 bis 4, wobei der Zustand über den Lebermetabolismus charakterisiert wird und die Gruppe von Bestandteilen eine Lebermetabolismusgruppe ist.

9. Ein Verfahren gemäß irgendeinem der Ansprüche 1 bis 4, wobei der biologische Zustand eine Toxizität ist und die Gruppe von Bestandteilen eine Toxizitätsgruppe ist.

10. Ein Verfahren gemäß Anspruch 9, wobei die Toxizität eine Nebenreaktion auf ein Arzneimittel ist.

11. Ein Verfahren wie in Anspruch 1 beansprucht, wobei der erste Profildatensatz wenigstens 5 Elemente umfasst.

## Revendications

1. Procédé pour évaluer un état biologique chez un sujet, basé sur un premier échantillon obtenu du sujet, l'échantillon fournissant une source d'ARN ; lequel procédé est **caractérisé en ce qu'**il comprend :
(a) l'opération consistant à prélever sur le premier échantillon, un premier ensemble de données de profil, lequel premier ensemble de données de profil comprend au moins 3 éléments, chaque élément étant une mesure quantitative relative de la quantité, dans ledit premier échantillon, d'un constituant d'ARN transcrit distinct dans un panel de constituants choisis de façon que la mesure desdits constituants permette une évaluation dudit état biologique, la quantification relative d'ARNm dans ledit échantillon étant déterminée par mise en oeuvre d'une PCR quantitative sur l'ARN extrait de l'échantillon avec une efficacité définie d'amplification pour les produits de transcription cibles, et détermination de la différence de cycles de seuil (delta C_{T}) entre un marqueur de taille et les produits de transcription cibles auxquels on s'intéresse ; et
(b) la production d'un ensemble de données de profil étalonné pour le panel, chaque élément de l'ensemble de données de profil étalonné étant fonction d'un élément correspondant du premier ensemble de données de profil et d'un élément correspondant d'un ensemble de données de profil de base pour le panel ; où l'ensemble de données de profil étalonné représente un ensemble de valeurs dans lequel un motif de variation est en corrélation d'une manière reproductible avec un état particulier.

2. Procédé selon la revendication 1, comprenant en outre la distinction entre deux états biologiques par mise en oeuvre de (a) et (b) pour chacun des deux états biologiques.

3. Procédé selon la revendication 1, dans lequel la fonction comprend un rapport de tels éléments.

4. Procédé selon la revendication 3, dans lequel la fonction comprend un logarithme du rapport.

5. Procédé selon l'une quelconque des revendications 1 à 4, dans lequel l'état biologique est un processus morbide complexe mettant en jeu de multiples gènes, la maladie étant d'un type impliquant au moins l'un parmi une inflammation, une maladie auto-immune, une maladie dégénérative, une allergie, une maladie vasculaire, une ischémie, un cancer, une maladie développementale, un état hormonal, un vieillissement et une maladie infectieuse.

6. Procédé selon la revendication 5, dans lequel l'état est **caractérisé par** une inflammation et le panel comprend au moins l'un parmi Il-1alpha, Il-1bêta, Il-2, Il-4, Il-5, Il-6, Il-8, Il-10, I12p40, Il-18, GMCSF, IFN-gamma, TGF-b, cox-1, cox-2, ICE, mmp-3, mmp-9, ICAM, TNF-a et TNF-b.

7. Procédé selon la revendication 1, dans lequel l'échantillon est un échantillon de sang.

8. Procédé selon l'une quelconque des revendications 1 à 4, dans lequel l'état est **caractérisé par** un métabolisme hépatique et le panel de constituants est un panel de métabolismes hépatiques.

9. Procédé selon l'une quelconque des revendications 1 à 4, dans lequel l'état biologique est une toxicité et le panel de constituants est un panel de toxicités.

10. Procédé selon la revendication 9, dans lequel la toxicité est une réaction adverse à un médicament.

11. Procédé selon la revendication 1, dans lequel ledit premier ensemble de données de profil comprend au moins 5 éléments.
